(19) Europäisches Patentamt
European Patent Office
Office européen des brevets

(11) EP 4 768 483 A1

(12) **EUROPEAN PATENT APPLICATION**
published in accordance with Art. 153(4) EPC

(43) Date of publication:
**01.07.2026 Bulletin 2026/27**

(21) Application number: **24870458.7**

(22) Date of filing: **12.09.2024**

(51) International Patent Classification (IPC):
***C07D 417/14*** (2006.01) ***C07D 419/14*** (2006.01)
***C07D 417/06*** (2006.01) ***C07D 403/14*** (2006.01)
***A61K 31/404*** (2006.01) ***A61K 31/426*** (2006.01)
***A61K 31/4427*** (2006.01) ***A61P 35/00*** (2006.01)

(52) Cooperative Patent Classification (CPC):
**A61K 31/404; A61K 31/426; A61K 31/427; A61K 31/4427; A61P 1/00; A61P 3/04; A61P 7/00; A61P 35/00; A61P 37/02; C07D 403/14; C07D 417/06; C07D 417/14; C07D 419/14**

(86) International application number:
**PCT/CN2024/118483**

(87) International publication number:
**WO 2025/066914 (03.04.2025 Gazette 2025/14)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **28.09.2023 CN 202311273015**

(71) Applicant: **Arigenx Therapeutics Co., Ltd.**
**Shenzhen, Guangdong 518083 (CN)**

(72) Inventor: **ZHANG, Suoming**
**Shenzhen, Guangdong 518083 (CN)**

(74) Representative: **Valet Patent Services Limited**
**c/o Caya 83713X**
**Am Börstig 5**
**96052 Bamberg (DE)**

(54) **AROMATIC HYDROCARBON RECEPTOR MODULATOR AND USE THEREOF**

(57) Disclosed in the present invention are an aromatic hydrocarbon receptor modulator, which has the following general formula (I):

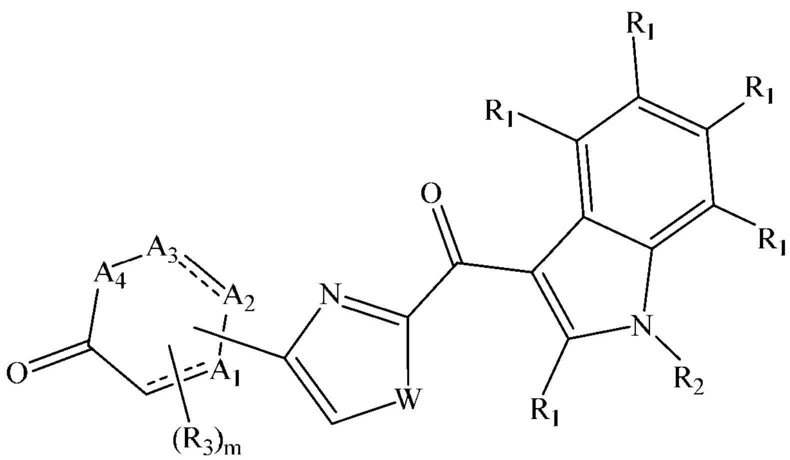

(I),

W is C=C or S, $A_1$, $A_2$, $A_3$ are independently a connecting key or C, and m is an integer between 1 to 7; each $R_1$ and $R_3$ are independently H, D, halogen, CN, OR, SR, N(R)R, - C(O)R, -C(O)OR, RC(O)O-, $S(O)_nR$, unsubstituted or 1 to 6 $R_a$ substituted C1-C8 alkyl, C2-C8 alkenyl, C2-C8 alkynyl or C3-C10 cycloalkyl; $A_4$ is independently O or $NR_4$; $R_2$ is H, D or

EP 4 768 483 A1

$-(CH_2O)_aC(O)(CH_2)_b(O)_c(CH(R_c))_d(CH_2CH_2O)_eR_c$; $R_c$ is independently H, D, C1-C8 alkyl, $NH_2$-, (C1-C8 alkyl)NH-, (C1-C8 alkyl)$_2$N-, $NH_2$(C1-C8)alkyl-. The aromatic hydrocarbon receptor modulator of the present invention has good activity in a drug for treating cancer and obesity, or in immunoregulation, hematopoiesis, cell cycle, or intestinal barrier.

## Description

### TECHNICAL FIELD

The present invention generally relates to the technical field of pharmaceutical synthesis, and in particular, to an aryl hydrocarbon receptor modulator and a use thereof.

### BACKGROUND

The aryl hydrocarbon receptor (AHR) is a member of the bHLH-PER-ARNT-SIM (bHLH-PAS) subfamily within the basic helix-loop-helix (bHLH) superfamily. The AHR is the only ligand-activatable receptor in the bHLH-PAS family [Murray et al., Nat. Rev. Cancer, 2014, 14(12), 801-814; Bersten et al., Nat. Rev. Cancer, 2013, 13(12), 827-841].

Agonists of the AHR are highly diverse, including traditionally recognized aromatic hydrocarbon xenobiotics, such as 2,3,7,8-tetrachlorodibenzo-p-dioxin (TCDD). Over the past two decades, numerous endogenous agonists have also been gradually identified, such as 2-(1'H-indole-3'-carbonyl)-thiazole-4-carboxylic acid methyl ester (ITE) and the tryptophan metabolite kynurenic acid. The agonists of the AHR can also be derived from dietary intake, such as indole-3-carbinol (I3C) from cruciferous vegetables, and metabolites (e.g., indoleacetic acid (IAA)) from the gut microbiota. After activation by the agonist, the AHR translocate from the cytoplasm into the nucleus and forms a heterodimer with the aryl hydrocarbon receptor nuclear translocator (ARNT). The heterodimer binds to the xenobiotic response element (XRE) in DNA, thereby initiating transcription of XRE-regulated genes (e.g., genes encoding cytochrome CYP450 enzymes (e.g., CYP1A1)). The AHR can also regulate XRE-independent gene expression and metabolism through protein-protein interactions, for example, with the estrogen receptor.

The AHR pathway regulates numerous key innate and adaptive immune responses. Studies have found that some AHR agonists promote T-helper 17 (Th17) cell differentiation and interleukin-17 (IL-17) secretion. In contrast, other AHR agonists can induce the lateral differentiation of Th17 cells into regulatory T (Treg) cells and enhance the suppressive activity of Treg cells [Quintana et al., Nature, 2008, 453(7191), 65-71; Mezrich et al., J. Immunol., 2010, 185(6), 3190-3198]. Studies have demonstrated that AHR activation can inhibit macrophage-regulated innate inflammatory responses (e.g., reducing lipopolysaccharide (LPS)-induced expression of IL-1b, IL-6, IL-12, and TNFalpha) and inhibit dendritic cells (reducing dendritic cell activation and promoting IL-10 expression) [Kimura et al., J. Exp. Med., 2009, 206(9), 2027-2035; Wang et al., Clin. Exp. Immunol., 2014, 177(2), 521-530; Wei et al., Lab. Invest., 2014, 94(5), 528-535; Nguyen et al., Proc. Natl. Acad. Sci. USA, 2010, 107(46), 19961-19966].

As an important key factor in immune regulation, the AHR is considered a key target for immune-related diseases. Studies have indicated that the AHR plays a regulatory role in models of septic shock, herpes simplex virus ocular infection, and Toxoplasma gondii infection. The agonists of the AHR also exhibit protective effects in immune diseases such as multiple sclerosis, inflammatory bowel disease, rheumatoid arthritis, and psoriasis. The immune-regulatory activity associated with the AHR also plays an important role in numerous inflammation-related metabolic diseases, such as atherosclerosis, type I diabetes, chronic kidney disease, bone metabolism-related diseases, and pulmonary arterial hypertension. Therefore, it is of great significance to develop novel agonists targeting the AHR for providing new drugs for the treatment of immune-related diseases.

### SUMMARY

One aspect of the present invention provides an aryl hydrocarbon receptor modulator or an enantiomer, a prodrug, or a pharmaceutically acceptable salt thereof, including the following general formula (I):

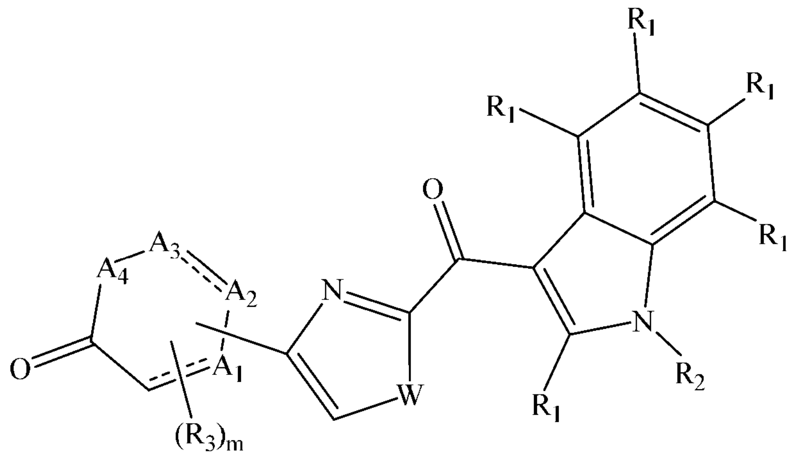

(I),

wherein

W is C=C or S;
$A_1$, $A_2$, and $A_3$ are each independently a bond or C; m is an integer from 1 to 7; $R_3$ is independently H, D, halogen, CN, OR, SR, N(R)R, -C(O)R, -C(O)OR, RC(O)O-, $S(O)_nR$, unsubstituted C1-C8 alkyl or substituted C1-C8 alkyl with 1-6 $R_a$, unsubstituted C2-C8 alkenyl or substituted C2-C8 alkenyl with 1-6 $R_a$, unsubstituted C2-C8 alkynyl or substituted C2-C8 alkynyl with 1-6 $R_a$, or unsubstituted C3-C10 cycloalkyl or substituted C3-C10 cycloalkyl with 1-6 $R_a$; and $R_a$ is D, halogen, OR, SR, N(R)R, -C(O)R, -C(O)OR, RC(O)O-, or $S(O)_nR$;
$A_4$ is independently O or $NR_4$; $R_4$ is H, unsubstituted C1-C8 alkyl or substituted C1-C8 alkyl with 1-6 $R_b$, unsubstituted C2-C8 alkenyl or substituted C2-C8 alkenyl with 1-6 $R_b$, unsubstituted C2-C8 alkynyl or substituted C2-C8 alkynyl with 1-6 $R_b$, or unsubstituted C3-C10 cycloalkyl or substituted C3-C10 cycloalkyl with 1-6 $R_b$; and $R_b$ is D, halogen, OR, SR, N(R)R, -C(O)R, - C(O)OR, RC(O)O-, or $S(O)_nR$;
$R_1$ is independently H, D, halogen, CN, OR, SR, N(R)R, -C(O)R, -C(O)OR, RC(O)O-, $S(O)_nR$, unsubstituted C1-C8 alkyl or substituted C1-C8 alkyl with 1-6 $R_b$, unsubstituted C2-C8 alkenyl or substituted C2-C8 alkenyl with 1-6 $R_b$, unsubstituted C2-C8 alkynyl or substituted C2-C8 alkynyl with 1-6 $R_b$, or unsubstituted C3-C10 cycloalkyl or substituted C3-C10 cycloalkyl with 1-6 $R_b$; Rb is D, halogen, OR, SR, N(R)R, -C(O)R, -C(O)OR, RC(O)O-, or $S(O)_nR$; or two adjacent or meta R1 groups form a 3-10 membered carbocycle or a 3- 10 membered heterocycle containing 1 to 3 heteroatoms selected from N, O, or S;

n is 1 or 2;
R is independently H, C1-C8 alkyl, C2-C8 alkenyl, C2-C8 alkynyl, or C3-C10 cycloalkyl; and
$R_2$ is H, $-(CH_2O)_aC(O)(CH_2)_b(O)_c(CH(R_c))_d(CH_2CH_2O)_eR_c$, $-(CH_2O)_aP(O)(R_p)_2$, or - $(CH_2O)_a(CH(R_c))_d(CH_2CH_2O)_eR_c$, $R_p$ is independently selected form $(CH_2)_b(O)_c(CH(R_c))_d(CH_2CH_2O)_eR_c$; $R_c$ is independently H, D, C1-C8 alkyl, $NH_2$-, (C1-C8 alkyl)NH-, $(C1\text{-}C8\ alkyl)_2N$-, $NH_2$(C1-C8 alkyl)-, OH, HO-(C1-C8 alkyl)-, or (C1-C8 alkyl)O-; a, b, c, and d are each independently 0 or 1; and e is an integer from 0 to 3.

In a preferred embodiment of the present invention, when W is S, the general formula (I) is a general formula (Ia):

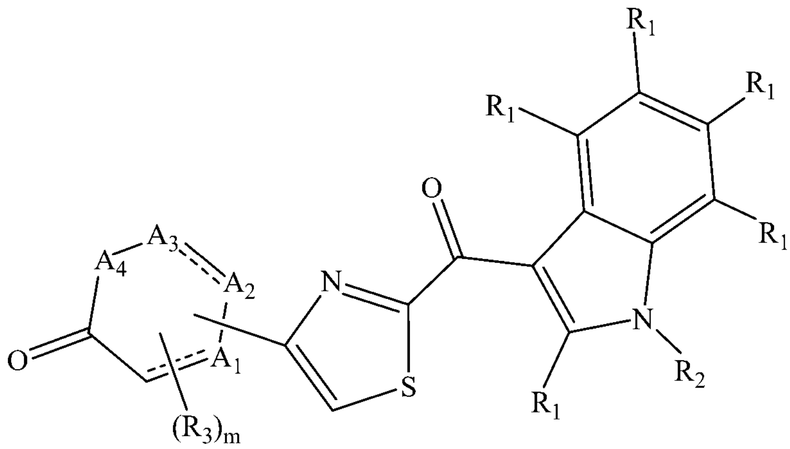

(Ia).

In a further preferred embodiment of the present invention, when $A_4$ is O, the general formula (Ia) is a general formula (Ia1):

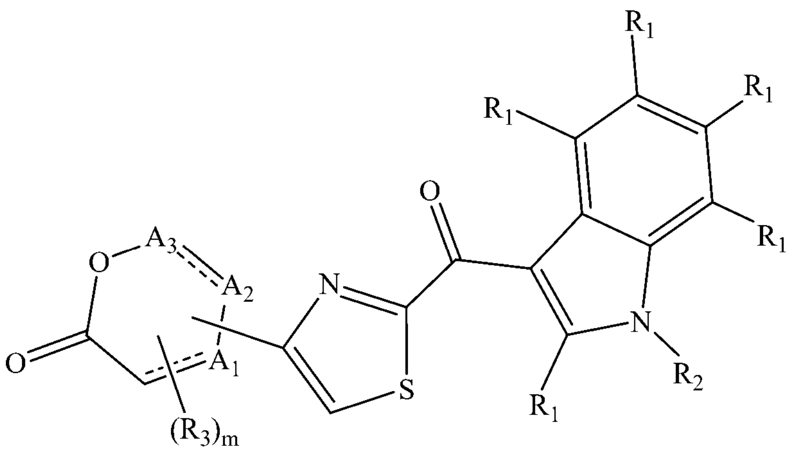

(Ia1);

or when $A_4$ is $NR_4$, the general formula (Ia) is a general formula (Ia2):

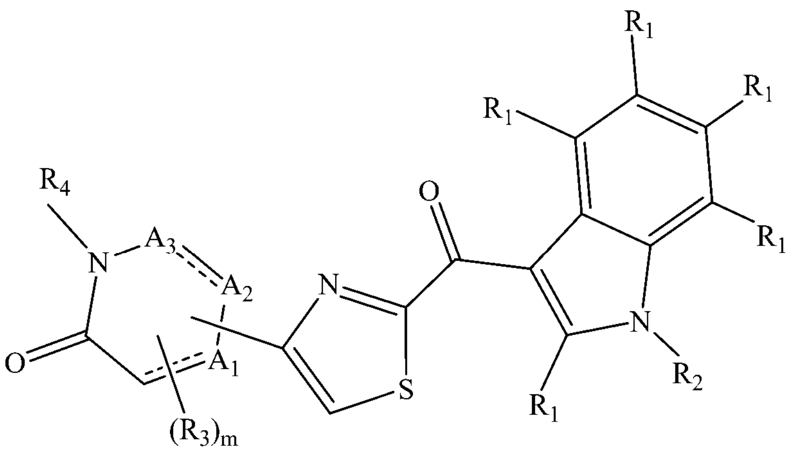

(Ia2).

In a preferred embodiment of the present invention, when W is C=C, the general formula (I) is a general formula (Ib):

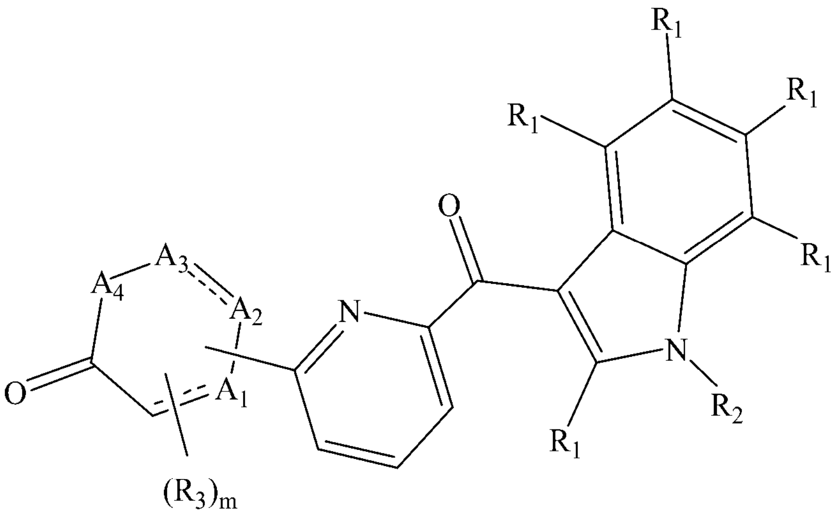

(Ib).

In a further preferred embodiment of the present invention, when $A_4$ is O, the general formula (Ib) is a general formula (Ib1):

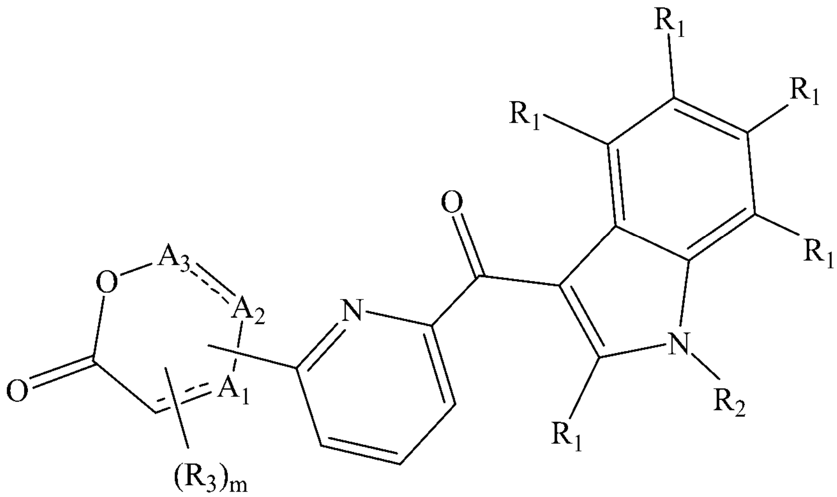

(Ib1);

or when $A_4$ is $NR_4$, the general formula (Ib) is a general formula (Ib2):

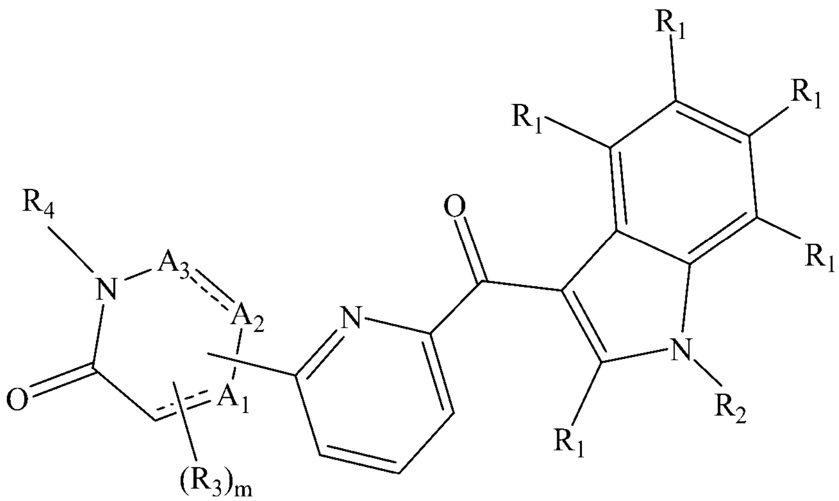

(Ib2).

In the general formula (I) of the present invention, in the structures "

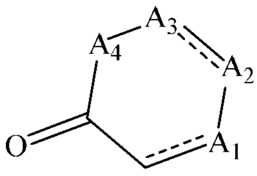 ", " 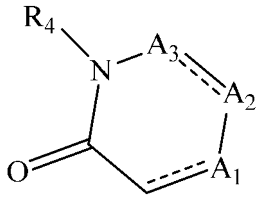 ", and " 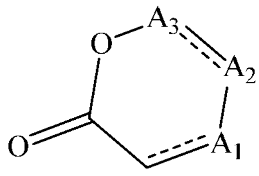 ",

the double dashed line bond between $A_2$ and $A_3$ indicates that the bond between $A_2$ and $A_3$ may be either a single bond or a double bond. Similarly, a double dashed bond between C and A1 indicates that the bond between C and A1 may be a single bond or a double bond. Furthermore, whether the bond between $A_2$ and $A_3$ is a single bond or a double bond is independent of whether the bond between C and $A_1$ is a single bond or a double bond, and there is no necessary correlation between the two.

In the general formula (I) of the present invention, a substituent

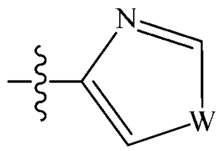

may be attached to any substitutable position on the structures "

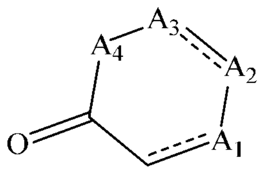 ", "

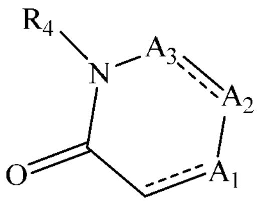 ", " 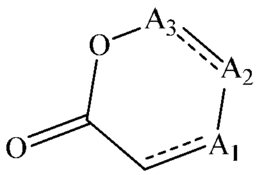 ",

i.e., the substituent may be attached to C, or may be attached to $A_1$, $A_2$, or $A_3$. There may be one or more $R_3$ groups. Two $R_3$ groups may be attached to the same atom or to different atoms, which is not particularly limited in the present invention.

In a preferred embodiment of the present invention,

$A_1$, $A_2$, and $A_3$ are each independently a bond or C; m is an integer from 1 to 5; $R_3$ is independently H, D, halogen, CN, OR, SR, N(R)R, -C(O)R, -C(O)OR, RC(O)O-, $S(O)_nR$, unsubstituted C1-C6 alkyl or substituted C1-C6 alkyl with 1-3 $R_a$, unsubstituted C2-C6 alkenyl OR substituted C2-C6 alkenyl with 1-3 $R_a$, unsubstituted C2-C6 alkynyl or substituted C2-C6 alkynyl with 1-3 $R_a$, or unsubstituted C3-C8 cycloalkyl or substituted C3-C8 cycloalkyl with 1-3 $R_a$; and $R_a$ is D, halogen, OR, SR, N(R)R, -C(O)R, -C(O)OR, RC(O)O-, or $S(O)_nR$;

n is 1 or 2; and

R is independently H, C1-C6 alkyl, C2-C6 alkenyl, C2-C6 alkynyl, or C3-C8 cycloalkyl; preferably,

$A_1$, $A_2$, and $A_3$ are each independently a bond or C; m is an integer from 1 to 3; $R_3$ is independently H, D, halogen, CN, OR, SR, N(R)R, -C(O)R, -C(O)OR, RC(O)O-, $S(O)_nR$, unsubstituted C1-C4 alkyl or substituted C1-C4 alkyl with 1-2 $R_a$, unsubstituted C2-C4 alkenyl or substituted C2-C4 alkenyl with 1-2 $R_a$, unsubstituted C2-C4 alkynyl or substituted C2-C4 alkynyl with 1-2 $R_a$, or unsubstituted C3-C7 cycloalkyl or substituted C3-C7 cycloalkyl with 1-2 $R_a$; and $R_a$ is D, halogen, OR, SR, N(R)R, -C(O)R, -C(O)OR, RC(O)O-, or $S(O)_nR$;

n is 1 or 2; and

R is independently H, C1-C4 alkyl, C2-C4 alkenyl, C2-C4 alkynyl, or C3-C7 cycloalkyl; more preferably,

$A_1$, $A_2$, and $A_3$ are each independently a bond or C; m is an integer from 1 to 2; $R_3$ is independently H, D, halogen, CN, OR, SR, N(R)R, -C(O)R, -C(O)OR, RC(O)O-, $S(O)_nR$, unsubstituted C1-C3 alkyl or substituted C1-C3 alkyl with 1 $R_a$, unsubstituted C2-C3 alkenyl or substituted C2-C3 alkenyl with 1 $R_a$, unsubstituted C2-C3 alkynyl or substituted C2-C3

alkynyl with 1 $R_a$, or unsubstituted C5-C6 cycloalkyl or substituted C5-C6 cycloalkyl with 1 $R_a$; and $R_a$ is D, halogen, OR, SR, N(R)R, -C(O)R, -C(O)OR, RC(O)O-, or $S(O)_nR$;
n is 1 or 2; and
R is independently H, C1-C3 alkyl, C2-C3 alkenyl, C2-C3 alkynyl, or C5-C6 cycloalkyl.

In another preferred embodiment of the present invention,

$R_1$ is independently H, D, halogen, CN, OR, SR, N(R)R, -C(O)R, -C(O)OR, RC(O)O-, $S(O)_nR$, unsubstituted C1-C6 alkyl or substituted C1-C6 alkyl with 1-3 $R_b$, unsubstituted C2-C6 alkenyl or substituted C2-C6 alkenyl with 1-3 $R_b$, unsubstituted C2-C6 alkynyl or substituted C2-C6 alkynyl with 1-3 $R_b$, or unsubstituted C3-C8 cycloalkyl or substituted C3-C8 cycloalkyl with 1-3 $R_b$; $R_b$ is D, halogen, OR, SR, N(R)R, -C(O)R, -C(O)OR, RC(O)O-, or $S(O)_nR$; or two adjacent or meta $R_1$ groups form a 3-8 membered carbocycle or a 3-8 membered heterocycle containing 1-3 heteroatoms selected from N, O, or S;
n is 1 or 2; and
R is independently H, C1-C6 alkyl, C2-C6 alkenyl, C2-C6 alkynyl, or C3-C8 cycloalkyl; preferably,
$R_1$ is independently H, D, halogen, CN, OR, SR, N(R)R, -C(O)R, -C(O)OR, RC(O)O-, $S(O)_nR$, unsubstituted C1-C4 alkyl or substituted C1-C4 alkyl with 1-2 $R_b$, unsubstituted C2-C4 alkenyl or substituted C2-C4 alkenyl with 1-2 $R_b$, unsubstituted C2-C4 alkynyl or substituted C2-C4 alkynyl with 1-2 $R_b$, or unsubstituted C3-C7 cycloalkyl or substituted C3-C7 cycloalkyl with 1-2 $R_b$; $R_b$ is D, halogen, OR, SR, N(R)R, -C(O)R, -C(O)OR, RC(O)O-, or $S(O)_nR$; or two adjacent or meta $R_1$ groups form a 3-7 membered carbocycle or a 3-7 membered heterocycle containing 1-2 heteroatoms selected from N, O, or S;
n is 1 or 2; and
R is independently H, C1-C4 alkyl, C2-C4 alkenyl, C2-C4 alkynyl, or C3-C7 cycloalkyl; more preferably,
$R_1$ is independently H, D, halogen, CN, OR, SR, N(R)R, -C(O)R, -C(O)OR, RC(O)O-, $S(O)_nR$, unsubstituted C1-C3 alkyl or substituted C1-C3 alkyl with 1 $R_b$, unsubstituted C2-C3 alkenyl or substituted C2-C3 alkenyl with 1 $R_b$, unsubstituted C2-C3 alkynyl or substituted C2-C3 alkynyl with 1 $R_b$, or unsubstituted C5-C6 cycloalkyl or substituted C5-C6 cycloalkyl with 1 $R_b$; $R_b$ is D, halogen, OR, SR, N(R)R, -C(O)R, -C(O)OR, RC(O)O-, or $S(O)_nR$; or two adjacent or meta $R_1$ groups form a 5-6 membered carbocycle or a 5-6 membered heterocycle containing 1-2 heteroatoms selected from N, O, or S;
n is 1 or 2; and
R is independently H, C1-C3 alkyl, C2-C3 alkenyl, C2-C3 alkynyl, or C5-C6 cycloalkyl.

In another preferred embodiment of the present invention,

$R_2$ is H, $-(CH_2O)_aC(O)(CH_2)_b(O)_c(CH(R_c))_d(CH_2CH_2O)_eR_c$, $-(CH_2O)_aP(O)(R_p)_2$, or $-(CH_2O)_a(CH(R_c))_d(CH_2CH_2O)_eR_c$, $R_p$ is independently selected from $(CH_2)_b(O)_c(CH(R_c))_d(CH_2CH_2O)_eR_c$; $R_c$ is independently H, D, C1-C6 alkyl, $NH_2$-, (C1-C6 alkyl)NH-, $(C1\text{-}C6\ alkyl)_2N$-, $NH_2$(C1-C6 alkyl)-, OH, HO-(C1-C6 alkyl)-, or (C1-C6 alkyl) O-; a, b, c, and d are each independently 0 or 1; and e is an integer from 0 to 2;
preferably,
$R_2$ is H, $-(CH_2O)_aC(O)(CH_2)_b(O)_c(CH(R_c))_d(CH_2CH_2O)_eR_c$, $-(CH_2O)_aP(O)(R_p)_2$, or $-(CH_2O)_a(CH(R_c))_d(CH_2CH_2O)_eR_c$; $R_p$ is independently selected from $(CH_2)_b(O)_c(CH(R_c))_d(CH_2CH_2O)_eR_c$; $R_c$ is independently H, D, C1-C4 alkyl, $NH_2$-, (C1-C4 alkyl)NH-, $(C1\text{-}C4\ alkyl)_2N$-, $NH_2$(C1-C4)alkyl-, OH, HO-(C1-C4)alkyl-, or (C1-C4 alkyl) O-; a, b, c, and d are each independently 0 or 1; and e is an integer of 0 or 1;
more preferably,
$R_2$ is H, $-(CH_2O)_aC(O)(CH_2)_b(O)_c(CH(R_c))_d(CH_2CH_2O)_eR_c$, $-(CH_2O)_aP(O)(R_p)_2$, or $-(CH_2O)_a(CH(R_c))_d(CH_2CH_2O)_eR_c$; $R_p$ is independently selected from $(CH_2)_b(O)_c(CH(R_c))_d(CH_2CH_2O)_eR_c$; $R_c$ is independently H, D, C1-C3 alkyl, $NH_2$-, (C1-C3 alkyl)NH-, $(C1\text{-}C3\ alkyl)_2N$-, $NH_2$(C1-C3)alkyl-, OH, HO-(C1-C3)alkyl-, or (C1-C3 alkyl) O-; a, b, c, and d are each independently 0 or 1; and e is an integer of 0 or 1.

In another preferred embodiment of the present invention,

$R_4$ is H, unsubstituted C1-C6 alkyl or substituted C1-C6 alkyl with 1-3 $R_b$, unsubstituted C2-C6 alkenyl or substituted C2-C6 alkenyl with 1-3 $R_b$, unsubstituted C2-C6 alkynyl or substituted C2-C6 alkynyl with 1-3 $R_b$, or unsubstituted C3-C8 cycloalkyl or substituted C3-C8 cycloalkyl with 1-3 $R_b$; and $R_b$ is D, halogen, OR, SR, N(R)R, -C(O)R, -C(O)OR, RC(O)O-, or $S(O)_nR$; and
R is independently H, D, C1-C6 alkyl, C2-C6 alkenyl, C2-C6 alkynyl, or C3-C8 cycloalkyl; preferably,
$R_4$ is H, unsubstituted C1-C4 alkyl or substituted C1-C4 alkyl with 1-2 $R_b$, unsubstituted C2-C4 alkenyl or substituted C2-C4 alkenyl with 1-2 $R_b$, unsubstituted C2-C4 alkynyl or substituted C2-C4 alkynyl with 1-2 $R_b$, or unsubstituted C3-C7 cycloalkyl or substituted C3-C7 cycloalkyl with 1-2 $R_b$; and $R_b$ is D, halogen, OR, SR, N(R)R, -C(O)R, -C(O)OR, RC(O)O-, or $S(O)_nR$; and

R is independently H, D, C1-C4 alkyl, C2-C4 alkenyl, C2-C4 alkynyl, or C3-C7 cycloalkyl; more preferably,

$R_4$ is H, unsubstituted C1-C3 alkyl or substituted C1-C3 alkyl with 1 $R_b$, unsubstituted C2-C3 alkenyl or substituted C2-C3 alkenyl with 1 $R_b$, unsubstituted C2-C3 alkynyl or substituted C2-C3 alkynyl with 1 $R_b$, or unsubstituted C5-C6 cycloalkyl or substituted C5-C6 cycloalkyl with 1 $R_b$; and $R_b$ is D, halogen, OR, SR, N(R)R, -C(O)R, -C(O)OR, RC(O)O-, or $S(O)_nR$; and

R is independently H, D, C1-C3 alkyl, C2-C3 alkenyl, C2-C3 alkynyl, or C5-C6 cycloalkyl.

The aryl hydrocarbon receptor modulator of the present invention is the following compounds:

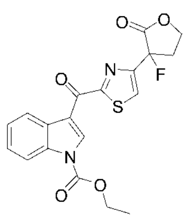
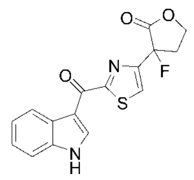
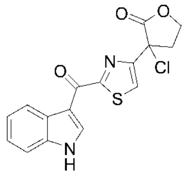
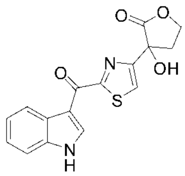
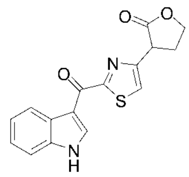
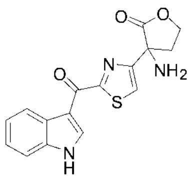
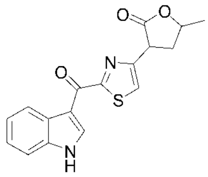
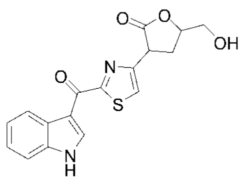
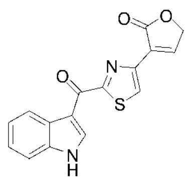
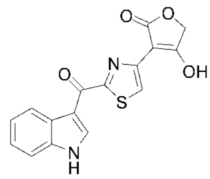
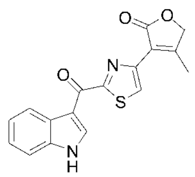
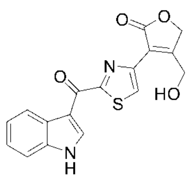
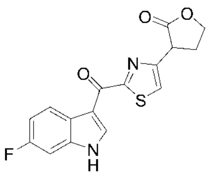
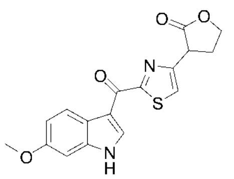
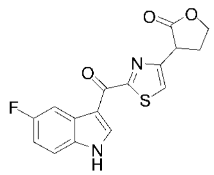
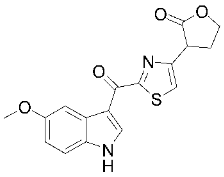
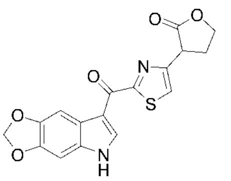
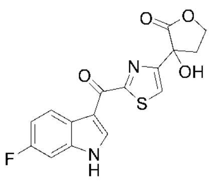
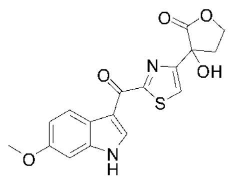
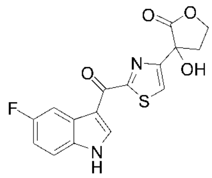
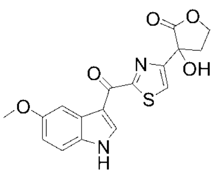
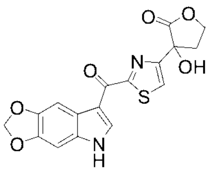
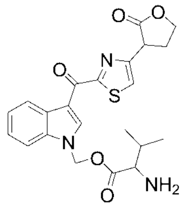
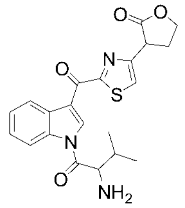
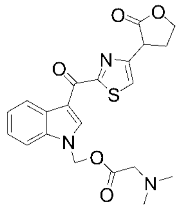

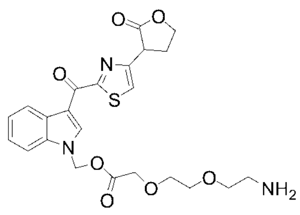
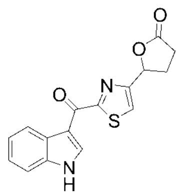
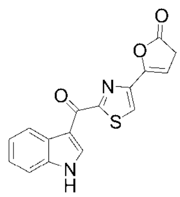
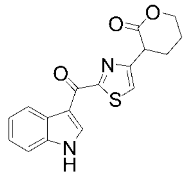
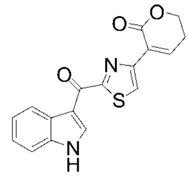

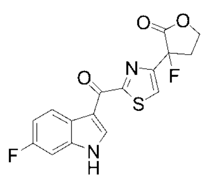
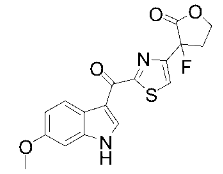
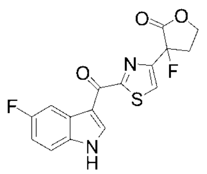
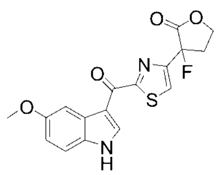
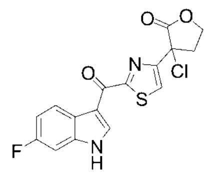

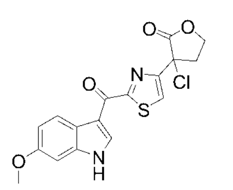
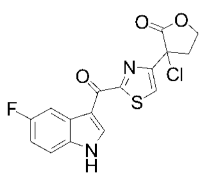
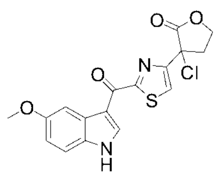
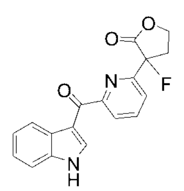
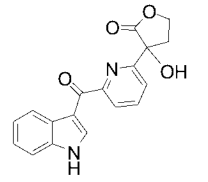

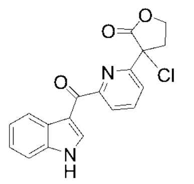
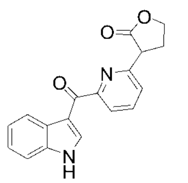
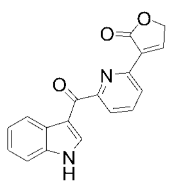
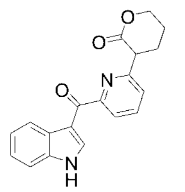
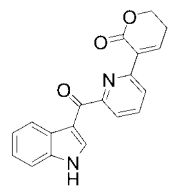

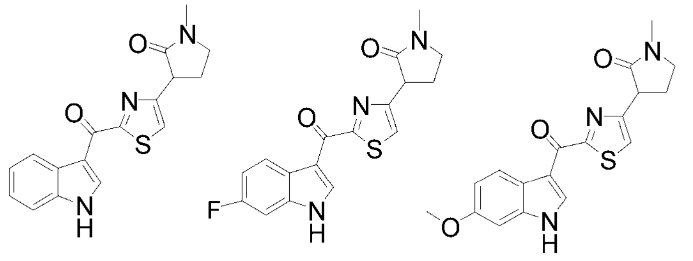
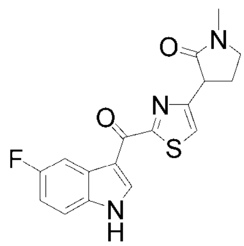
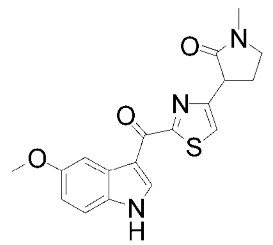

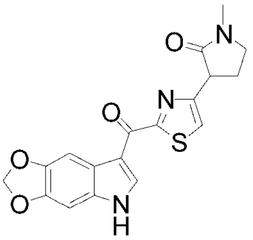
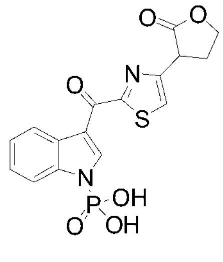
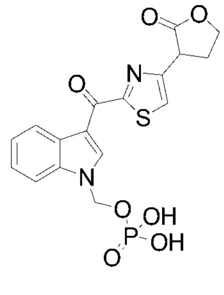
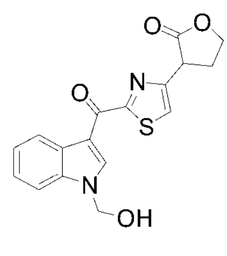

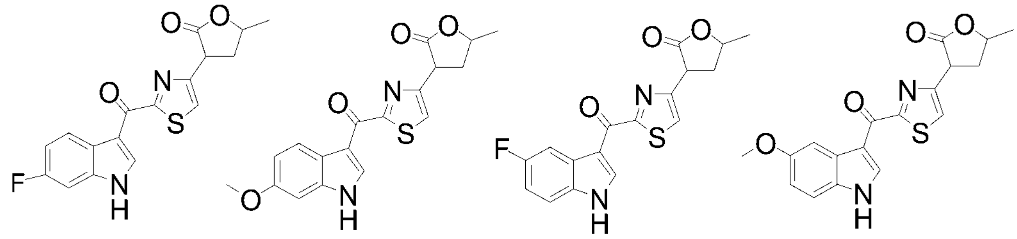

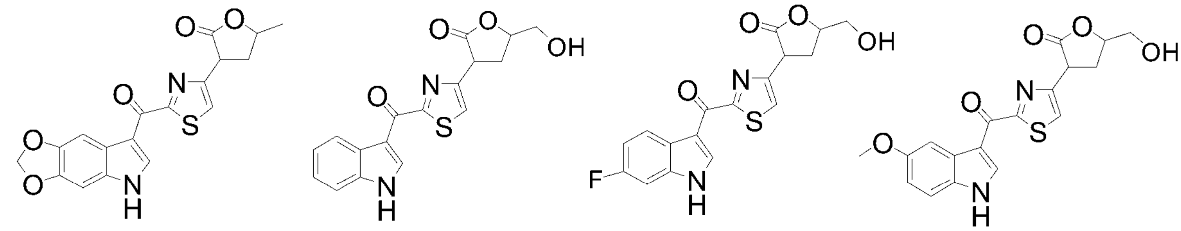

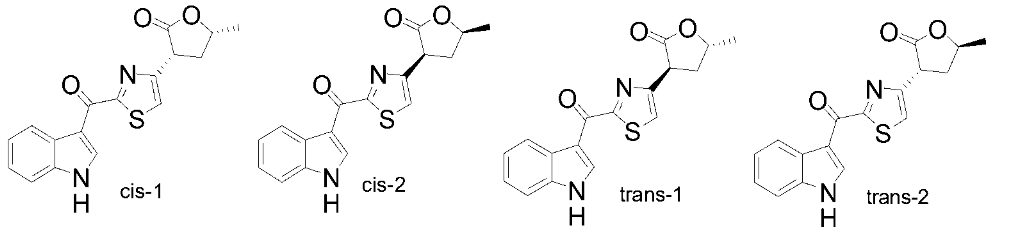

cis-1 cis-2 trans-1 trans-2

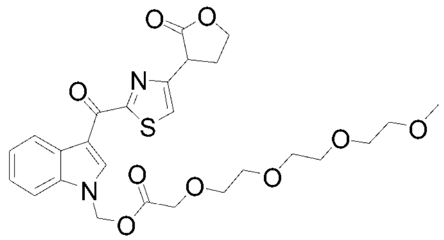

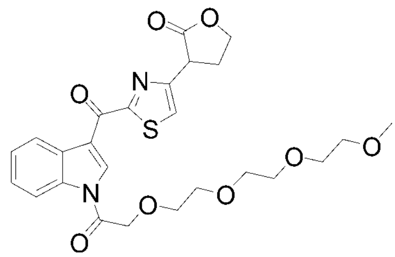

.

Another aspect of the present invention provides a salt, a prodrug, a hydrate, a solvate, or a deuterated derivative further substituted with deuterium of the aryl hydrocarbon receptor modulator of the general formula (I) of the present invention.

Another aspect of the present invention provides a use of the aryl hydrocarbon receptor modulator of the general formula (I) of the present invention in the preparation of a medicament for treating a central nervous system disease, cancer, or obesity, or in the preparation of a medicament for immunomodulation, hematopoiesis, cell cycle regulation, or intestinal barrier regulation; preferably, the central nervous system disease is selected from Alzheimer's disease, Parkinson's disease, and multiple sclerosis; the immunomodulation is selected from immunomodulation for psoriasis, atopic dermatitis, lupus erythematosus, or vitiligo; and the intestinal barrier regulation is for inflammatory bowel disease.

Another aspect of the present invention provides a use of the aryl hydrocarbon receptor modulator of the general formula (I) of the present invention in the treatment of a central nervous system disease, cancer, or obesity, or in immunomodulation, hematopoiesis, cell cycle regulation, or intestinal barrier regulation; preferably, the central nervous system disease is selected from Alzheimer's disease, Parkinson's disease, and multiple sclerosis; the immunomodulation is selected from immunomodulation for psoriasis, atopic dermatitis, lupus erythematosus, or vitiligo; and the intestinal barrier regulation is for inflammatory bowel disease.

A synthesis process of the aryl hydrocarbon receptor modulator of the general formula (I) of the present invention is as follows:

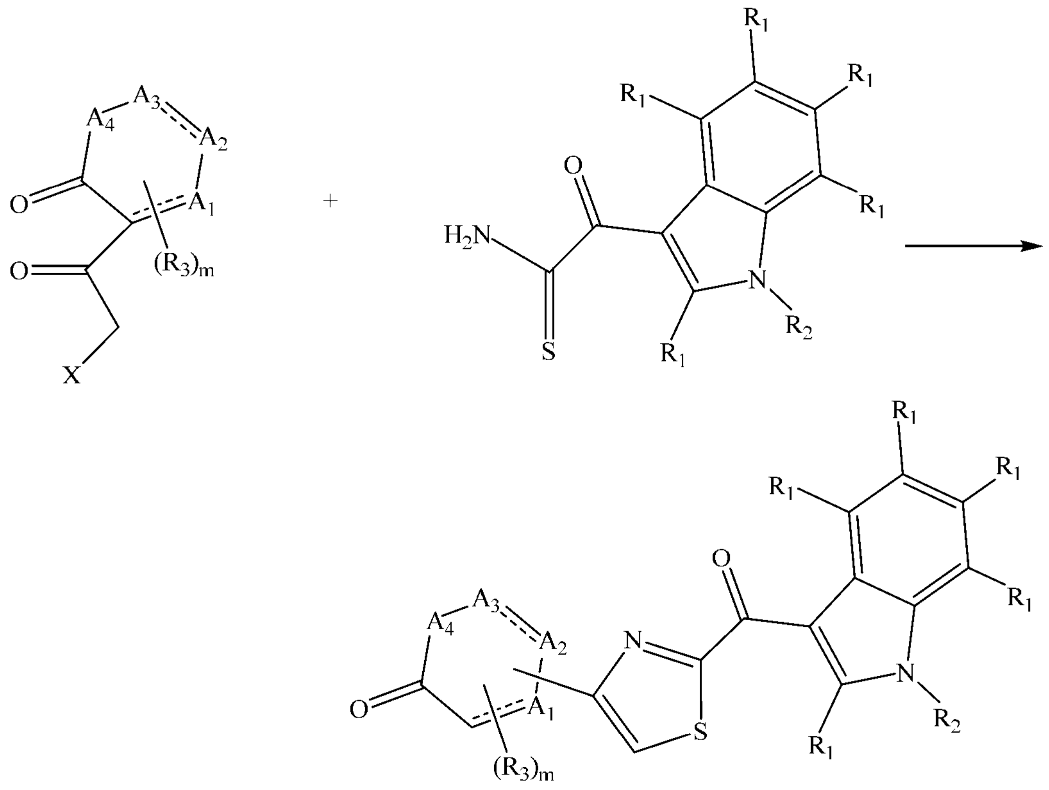

General formula (Ia),

wherein X is halogen.

DETAILED DESCRIPTION

SYNTHESIS OF INTERMEDIATE INT-1

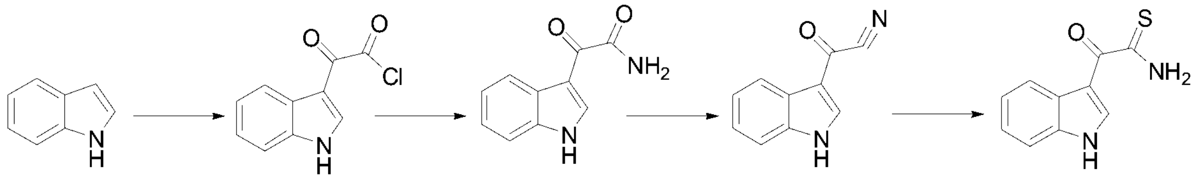

Int-1A Int-1B Int-1C Int-1

Step 1: 2-(1H-indol-3-yl)-2-oxalyl chloride (Int-1A)

At room temperature, indole (131 g, 1118 mmol) was dissolved in methyl tert-butyl ether (MTBE) (983 mL). The mixture was cooled to -10°C. Oxalyl chloride (149 g, 1174 mmol) was added dropwise slowly, and a temperature of -10°C to 5°C was maintained during the addition. After the addition was complete, the mixture was allowed to warm to room temperature naturally and reacted for 1 hour. Petroleum ether (983 mL) was added. The mixture was stirred at room temperature for 30 minutes and then filtered. The filter cake was washed with petroleum ether (200 mL). The solid filter cake was dried under reduced pressure to obtain a yellow solid Int-1A (228 g).

Step 2: 2-(1H-indol-3-yl)-2-oxalamide (Int-1B)

Aqueous ammonia (25%, 749 mL) and ethanol (1140 mL) were mixed and cooled to - 5°C to 14°C. The Int-1A (228 g, 1101 mmol) was then added slowly in portions. After the addition was complete, the reaction mixture was maintained at -5°C to 14°C for 2 hours. The reaction mixture was poured into water (1140 mL). The mixture was stirred at

room temperature for 30 minutes and then filtered. The filter cake was washed with water and dried in an oven at 60°C overnight to obtain a white solid product Int-1B (170 g).

Step 3: 1H-indole-3-carbonyl cyanide (Int-1C)

The Int-1B (170 g, 904 mmol) was dissolved in ethyl acetate (2000 mL). Pyridine (214 g, 2712 mmol) was added at room temperature. The mixture was cooled to 0°C to 5°C. Trifluoroacetic anhydride (284 g, 1356 mmol) was added dropwise slowly over 1.5 hours. The reaction mixture was then maintained at 5°C to 18°C for an additional 1.5 hours. After completion of the reaction, the mixture was quenched with a saturated aqueous sodium bicarbonate solution (1700 mL) and stirred for 10 minutes. The mixture was extracted with ethyl acetate twice. The combined organic phases were washed with 0.5 N dilute hydrochloric acid (650 mL × 2) and then washed with saturated brine (650 mL) once, dried over anhydrous sodium sulfate, and concentrated to dryness to obtain a crude product. The crude product was treated with a mixture of petroleum ether (PE) and ethyl acetate (EA) (PE/EA = 10/1, 400 mL) and filtered to obtain a brown solid Int-1C (145 g).

Step 4: Intermediate Int-1

Triethylamine (88.4 g, 873 mmol) was added to a solution of the Int-1C (135 g, 794 mmol) in pyridine (1350 mL) at 60°C. Ammonium sulfide (108 g, 1588 mmol) was then added. The reaction mixture was maintained at 60°C for 1.5 hours. The reaction mixture was then cooled to room temperature and slowly poured into 1 N hydrochloric acid (7 L). The mixture was extracted with ethyl acetate (2 L × 3). The combined organic phases were washed with saturated brine (2 L), dried over anhydrous sodium sulfate, filtered, and concentrated to dryness to obtain a crude product of the Int-1. The crude Int-1 was stirred and washed twice with a mixture of PE and EA (PE/EA = 10/1, 500 mL) and filtered to obtain a yellow solid Int-1 (90 g).

LCMS $[M+H]^+$ = 205.2.

$^1H$ NMR (400 MHz, DMSO-$d_6$) δ 12.15 (s, 1H), 10.23 (s, 1H), 10.02 (s, 1H), 8.20 (d, J= 3.2 Hz, 1H), 8.14 (dd, *J*= 5.8, 3.2 Hz, 1H), 7.58 -7.47 (m, 1H), 7.31 -7.19 (m, 2H).

TABLE 1 PREPARATION OF INTERMEDIATES INT-2 TO INT-9 BY THE SAME METHOD AS THAT FOR PREPARING INTERMEDIATE INT-1

| Intermediate | Starting Material | Structural Formula | LCMS (ESI): $[M+H]^+$ |
|---|---|---|---|
| Int-2 | 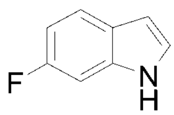 | 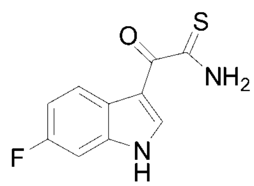 | 331 |
| Int-3 | 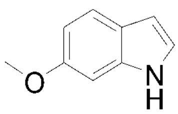 | 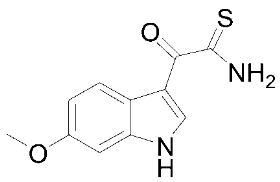 | 343 |
| Int-4 | 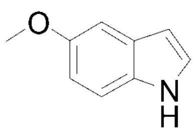 | 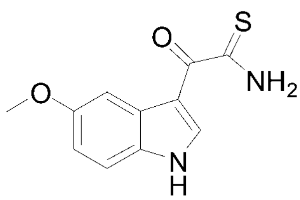 | 343 |
| Int-5 | 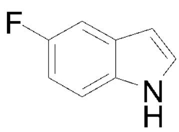 | 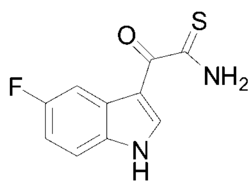 | 331 |

(continued)

| Intermediate | Starting Material | Structural Formula | LCMS (ESI): [M+H]+ |
|---|---|---|---|
| Int-6 | 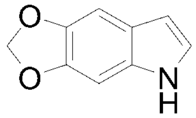 | 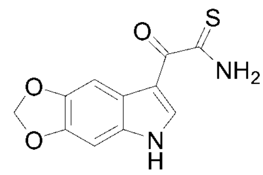 | 357 |
| Int-7 | 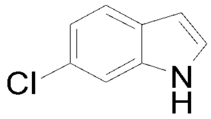 | 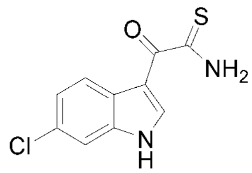 | 239 |
| Int-8 | 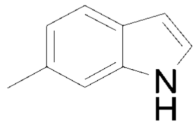 | 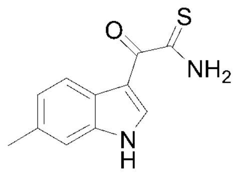 | 219 |
| Int-9 | 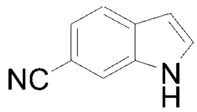 | 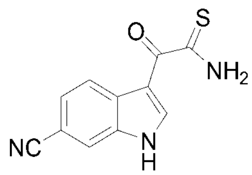 | 230 |

EXAMPLE 1 SYNTHESIS OF COMPOUND 1

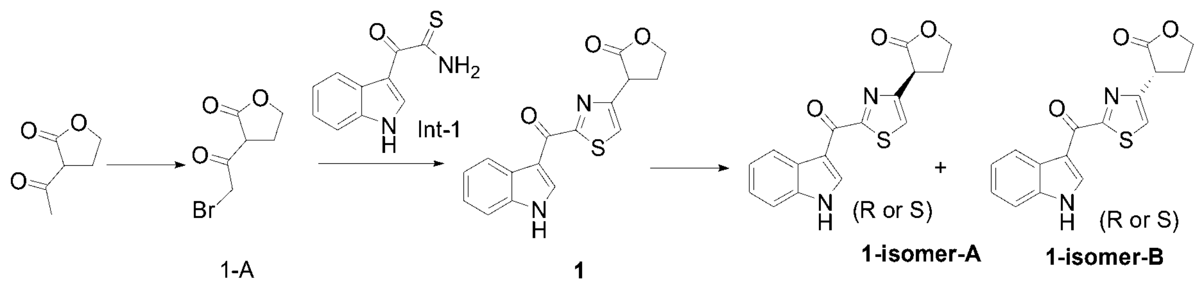


1-A    1    **1-isomer-A**    **1-isomer-B**

Step 1: Synthesis of Intermediate 1-A

At room temperature, acetic acid (2.40 g, 40.0 mmol, 1.00 eq.) was added to a solution of 2-acetylbutyrolactone (5.12 g, 40.0 mmol, 1.00 eq.) in dichloromethane (100 mL). The reaction mixture was cooled to below 0°C. A solution of bromine (12.8 g, 80.0 mmol, 2.00 eq.) in dichloromethane (50 mL) was added dropwise over 45 minutes. After the dropwise addition was complete, the reaction mixture was warmed to room temperature and stirred for 1 hour. The reaction mixture was filtered and the filtrate was concentrated under reduced pressure to obtain a brown oily intermediate 1-A (1.50 g, 18.2%), which was used directly in the next step.

Step 2: Synthesis of Compound 1

At room temperature, Intermediate 1-A (2.50 g, 12.1 mmol, 1.00 eq.) was added to a solution of intermediate Int-1 (3.00 g, 14.6 mmol, 1.20 eq.) in tetrahydrofuran (30 mL). The mixture was stirred overnight at room temperature in nitrogen atmosphere. The mixture was concentrated to dryness under reduced pressure. Ice water was added, followed by stirring, and a solid precipitated. The solid was filtered to obtain a crude product (2.50 g). The crude product was recrystallized from

ethyl acetate to obtain Compound 1 (a yellow solid, 2.30 g, a yield of 51%).

LCMS (ESI): $[M+H]^+$ = 313.0.

$^1H$ NMR (400 MHz, DMSO-$d_6$) δ 12.25 (s, 1H), 8.99 (d, *J* = 2.1 Hz, 1H), 8.38 -8.27 (m, 1H), 8.03 (s, 1H), 7.61 - 7.55 (m, 1H), 7.33 - 7.25 (m, 2H), 4.58-4.53 (m, 1H), 4.47 - 4.35 (m, 2H), 2.80 - 2.60 (m, 2H).

**Compound 1-isomer-A and Compound 1-isomer-B**

Compound 1 (800 mg) was subjected to chiral preparative separation to obtain Compound 1-isomer-A (340 mg, fraction 1, retention time of 1.163 minutes) and Compound 1-isomer-B (340 mg, fraction 2, retention time of 1.418 minutes).

Analytical method:

Instrument: UPCC (waters)
Column: Regis (R, R) Whelk-O1 (4.6×100 mm, 3.5 μm)
Column temperature: 40°C
Mobile phase: $CO_2$/MeOH [0.2% $NH_3$ (7M in MeOH)] = 45/55
Flow rate: 3 mL/min
Back pressure: 2000 psi
Preparation method:

Instrument: SFC-150mgm (waters)
Column: Regis (R, R) Whelk-O1 (25×250 mm, 10 μm)
Column temperature: 30°C
Mobile phase: $CO_2$/MeOH [0.2% $NH_3$ (7M in MeOH)] = 50/50
Flow rate: 100 mL/min
Back pressure: 100 bar
Detection wavelength: 214 nm

**Compound 1-isomer-A**

LCMS (ESI): $[M+H]^+$ = 313.0.

$^1H$ NMR (400 MHz, MeOH-$d_4$) δ 9.04 (s, 1H), 8.38-8.35 (m, 1H), 7.82 (s, 1H), 7.52 - 7.49 (m, 1H), 7.28-7.26 (m, 2H), 4.64-4.59 (m, 1H), 4.50-4.44 (m, 1H), 4.32- 4.28 (m, 1H), 2.80 -2.60 (m, 2H).

**Compound 1-isomer-B**

LCMS (ESI): $[M+H]^+$ = 313.0.

$^1H$ NMR (400 MHz, MeOH-$d_4$) δ 9.04 (s, 1H), 8.38-8.35 (m, 1H), 7.82 (s, 1H), 7.52-7.49 (m, 1H), 7.28-7.26 (m, 2H), 4.64-4.59 (m, 1H), 4.50-4.44 (m, 1H), 4.32- 4.28 (m, 1H), 2.80-2.60 (m, 2H).

TABLE 2 PREPARATION OF COMPOUNDS 1-2 TO 1-9 BY THE SYNTHETIC METHOD FOR COMPOUND 1

| Compound | Starting Materia 1A | Starting Material B | Structural Formula | LCMS (ESI) and NMR |
|---|---|---|---|---|
| 1-2 | 1-A | Int-2 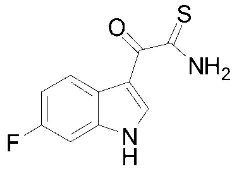 | 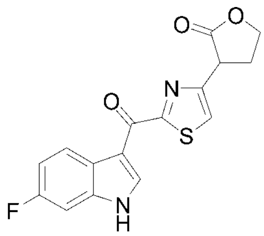 | LCMS (ESI):331[M+H]$^+$ $^1$H NMR (400 MHz, DMSO-*d*$_6$) $\delta$ 12.27 (d, *J* = 1.0 Hz, 1H), 8.99 (d, *J* = 3.2 Hz, 1H), 8.30 (dd, *J* = 8.7, 5.6 Hz, 1H), 8.05 (s, 1H), 7.39 (dd, *J* = 9.6, 2.3 Hz, 1H), 7.15 (dt, *J* = 1.4 Hz, 9.6 Hz, 1H), 4.56 (dt, *J* = 3.3 Hz, 8.4 Hz, 1H), 4.47 - 4.33 (m, 2H), 2.83 - 2.57 (m, 2H). |
| 1-3 | 1-A | Int-3 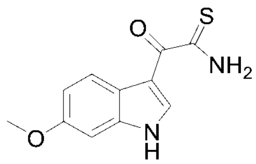 | 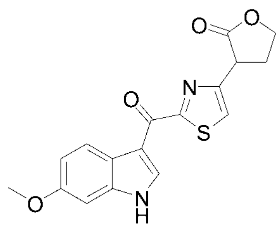 | LCMS (ESI):343[M+H]$^+$ $^1$H NMR (400 MHz, DMSO-*d*$_6$) $\delta$ 12.04 (s, 1H), 8.87 (d, *J* = 3.1 Hz, 1H), 8.16 (d, *J* = 8.7 Hz, 1H), 8.01 (s, 1H), 7.06 (d, *J* = 2.1 Hz, 1H), 6.91 (dd, *J* = 8.7, 2.2 Hz, 1H), 4.55 (td, *J* = 8.4, 3.3 Hz, 1H), 4.44-4.35 (m, 2H), 3.81 (s, 3H), 2.79 - 2.58 (m, 2H). |
| 1-4 | 1-A | Int-4 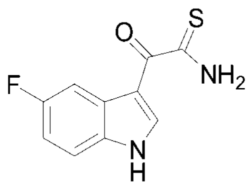 | 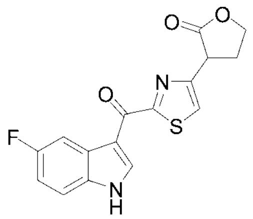 | LCMS (ESI): 331[M+H]$^+$ $^1$H NMR (400 MHz, DMSO-*d*$_6$) $\delta$ 12.35 (s, 1H), 9.03 (d, *J* = 3.3 Hz, 1H), 8.05 (s, 1H), 7.98 (dd, *J* = 9.8, 2.6 Hz, 1H), 7.60 (dd, *J* = 8.9, 4.6 Hz, 1H), 7.16 (td, *J* = 9.2, 2.6 Hz, 1H), 4.56 (td, *J* = 8.4, 3.3 Hz, 1H), 4.46 - 4.32 (m, 2H), 2.79-2.61 (m, 2H). |
| 1-5 | 1-A | Int-5 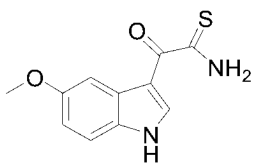 | 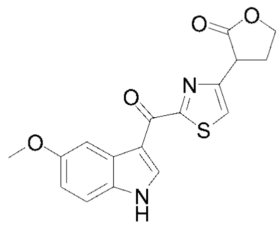 | LCMS (ESI): 343[M+H]$^+$ $^1$H NMR (400 MHz, DMSO-*d*$_6$) $\delta$ 12.15 (s, 1H), 8.93 (d, *J=1.0* Hz, 1H), 8.02 (s, 1H), 7.84 (s, 1H), 7.47 (d, *J* = 8.7 Hz, 1H), 6.92 (d, *J* = 6.8 Hz, 1H), 4.57-4.53 (m, 1H), 4.47 - 4.27 (m, 2H), 3.82 (s, 3H), 2.84 - 2.58 (m, 2H). |
| 1-6 | 1-A | Int-6 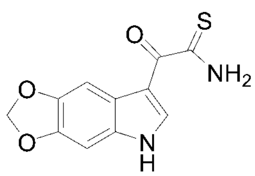 | 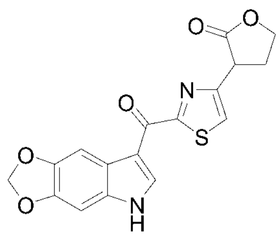 | LCMS (ESI): 357[M+H]$^+$ $^1$H NMR (400 MHz, DMSO-*d*$_6$) $\delta$ 12.05 (d, *J* = 1.0 Hz, 1H), 8.80 (d, *J* = 1.0 Hz, 1H), 8.01 (s, 1H), 7.70 (s, 1H), 7.10 (s, 1H), 6.04 (s, 2H), 4.55 (td, *J* = 8.4, 1.0 Hz, 1H), 4.46 - 4.26 (m, 2H), 2.91 - 2.55 (m, 2H). |

(continued)

| Compound | Starting Materia 1A | Starting Material B | Structural Formula | LCMS (ESI) and NMR |
|---|---|---|---|---|
| 1-7 | 1-A | Int-7 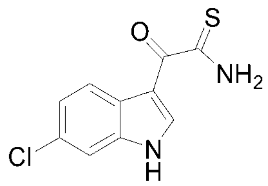 | 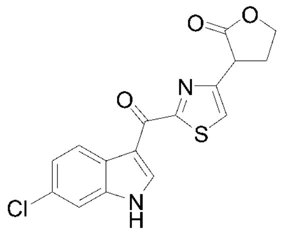 | LCMS (ESI): 347[M+H]$^+$ $^1$H NMR (400 MHz, DMSO-$d_6$) δ 12.04 (s, 1H), 8.99 (d, $J$ = 1.0 Hz, 1H), 8.36 (d, $J$ = 8.7 Hz, 1H), 8.00 (s, 1H), 7.36 (d, $J$ = 2.1 Hz, 1H), 7.20 (dd, $J$ = 8.7, 2.2 Hz, 1H), 4.55 (td, $J$ = 8.4, 3.3 Hz, 1H), 4.43-4.35 (m, 2H), 2.79 -2.58 (m, 2H). |
| 1-8 | 1-A | Int-8 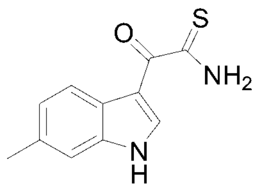 | 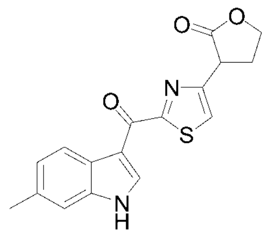 | LCMS (ESI):327[M+H]$^+$ $^1$H NMR (400 MHz, DMSO-$d_6$) δ 12.04 (s, 1H), 8.87 (d, $J$ = 3.1 Hz, 1H), 8.16 (d, $J$ = 8.7 Hz, 1H), 8.01 (s, 1H), 7.06 (d, $J$ = 2.1 Hz, 1H), 6.91 (dd, $J$ = 8.7, 2.2 Hz, 1H), 4.55 (td, J = 8.4, 3.3 Hz, 1H), 4.44-4.35 (m, 2H), 2.79 -2.58 (m, 2H).2.11 (s, 3H). |
| 1-9 | 1-A | Int-9 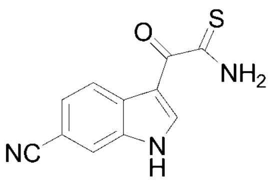 | 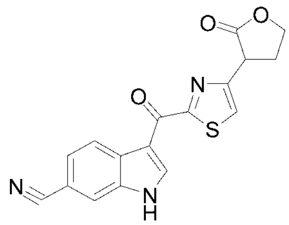 | LCMS (ESI):338[M+H]$^+$. |

**EXAMPLE 2 SYNTHESIS OF COMPOUND 2**

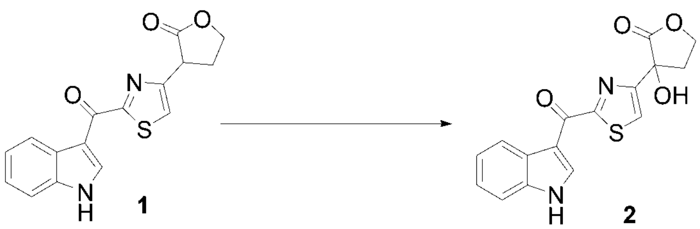

1 2

At room temperature, 1,5,7-Triazabicyclo [4.4.0] dec-5-ene (TBD, 5.00 mg, 0.032 mmol, 0.10 eq.) was added to a solution of Compound 1 (0.10 g, 0.32 mmol, 1.00 eq.) in N, N-dimethylformamide (DMF, 5 mL). The solution was warmed to 45°C and stirred overnight with the reaction vessel open. The reaction mixture was concentrated to dryness under reduced pressure, followed by purification by silica gel column chromatography (ethyl acetate: petroleum ether = 0% to 50%) to obtain Compound 2 (a yellow solid, 70 mg, a yield of 70%).

LCMS (ESI): [M+H]$^+$ = 329.0.

$^1$H NMR (400 MHz, DMSO-$d_6$) δ 12.26 (s, 1H), 8.93 (d, $J$ = 3.3 Hz, 1H), 8.31 (dd, $J$ = 5.9, 3.2 Hz, 1H), 8.09 (s, 1H), 7.68-7.50 (m, 1H), 7.40-7.20 (m, 2H), 6.98 (s, 1H), 4.64-4.43 (m, 2H), 2.94-2.88 (m, 1H), 2.62-2.52 (m, 1H).

TABLE 3 PREPARATION OF COMPOUNDS 2-2 TO 2-5 BY THE SYNTHETIC METHOD FOR COMPOUND 2 IN EXAMPLE 2

| Compound | Starting Material | Structural Formula | LCMS (ESI):[M+H]+ |
|---|---|---|---|
| 2-2 | Compound 1-2 | 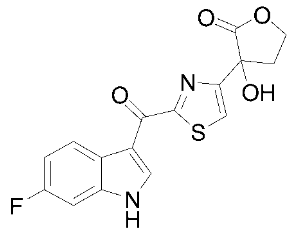 | LCMS (ESI): 347[M+H]+ <br> $^1$H NMR (400 MHz, DMSO-$d_6$) $\delta$ 12.29 (d, *J* = 1.0 Hz, 1H), 8.99 (d, *J* = 3.2 Hz, 1H), 8.31 (dd, *J* = 8.7, 5.6 Hz, 1H), 810 (s, 1H), 7.39 (dd, J = 9.6, 2.3 Hz, 1H), 7.15 (dt, *J* = 1.4 Hz, 9.6 Hz, 1H), 6.99 (s, 1H), 4.64-4.43 (m, 2H), 2.94-2.88 (m, 1H), 2.62-2.52 (m, 1H) |
| 2-3 | Compound 1-3 | 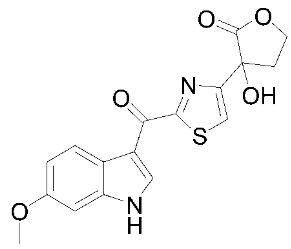 | LCMS (ESI): 359[M+H]+ |
| 2-4 | Compound 1-4 | 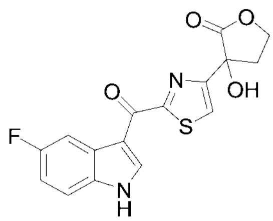 | LCMS (ESI): 347[M+H]+ |
| 2-5 | Compound 1-5 | 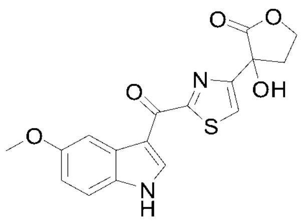 | LCMS (ESI): 359[M+H]+ |

**EXAMPLE 3 SYNTHESIS OF COMPOUND 3**

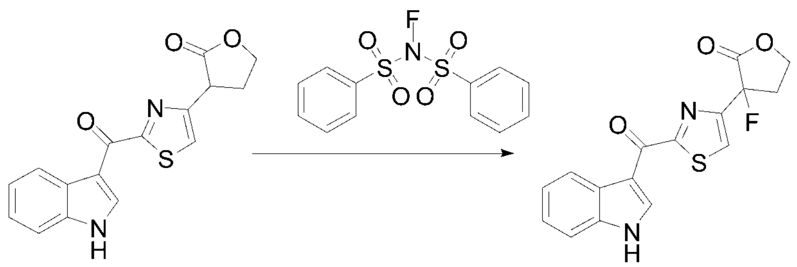

1 3

In nitrogen atmosphere, NaH (60.0 mg, 2.50 mmol, 2.50 eq.) was added to a solution of Compound 1 (0.312 g, 1.00 mmol, 1.00 eq.) in tetrahydrofuran (5 mL) at room temperature. After stirring for half an hour, N-fluorobenzenesulfonimide (378 mg, 1.20 mmol, 1.20 eq.) was added, and the mixture was stirred at room temperature for 1 hour. The mixture was concentrated to dryness under reduced pressure and purified by silica gel column chromatography (methanol: dichloromethane = 0% to 100%) to obtain Compound 3 (a yellow solid, 40 mg, 10%).

LCMS (ESI): $[M+H]^+$ = 331.0.

$^1H$ NMR (400 MHz, DMSO-$d_6$) δ 12.31 (s, 1H), 8.97 (s, 1H), 8.41 (s, 1H), 8.34 -8.11 (m, 1H), 7.59-7.56 (m, 1H), 7.41-7.08 (m, 2H), 4.70-4.64 (m, 1H), 4.62-4.41 (m, 1H), 3.29-3.19 (m, 1H), 3.10-2.93 (m, 1H).

TABLE 4 PREPARATION OF COMPOUNDS 3-2 TO 3-5 BY THE SYNTHETIC METHOD FOR COMPOUND 3 IN EXAMPLE 3

| Compound | Starting Material | Structural Formula | LCMS (ESI):$[M+H]^+$ |
|---|---|---|---|
| 3-2 | Compound 1-2 | 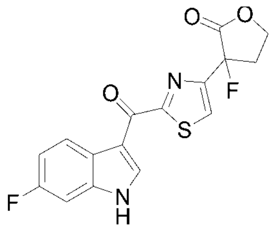 | LCMS (ESI):349$[M+H]^+$ |
| 3-3 | Compound 1-3 | 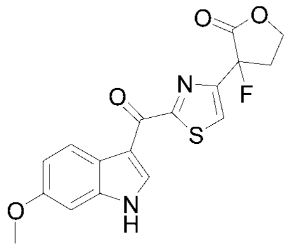 | LCMS (ESI): 361 $[M+H]^+$ |
| 3-4 | Compound 1-4 | 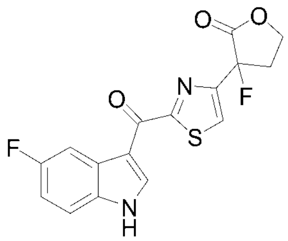 | LCMS (ESI): 349$[M+H]^+$ |
| 3-5 | Compound 1-5 | 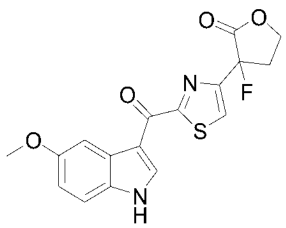 | LCMS (ESI): 361 $[M+H]^+$ |

**EXAMPLE 4 SYNTHESIS OF COMPOUND 4**

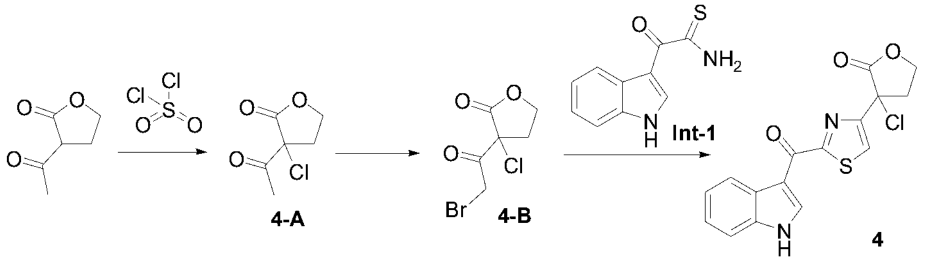

4-A 4-B Int-1 4

Step 1: Synthesis of Intermediate 4-A

In nitrogen atmosphere at 0°C, sulfuryl chloride (5.49 g, 41.0 mmol, 1.05 eq.) was added dropwise to alpha-acetyl-gamma-butyrolactone (5.00 g, 39.0 mmol, 1.00 eq.). The reaction mixture was stirred at room temperature for 5 hours. The mixture was poured into water (100 mL) and extracted with ethyl acetate (50 mL × 3). The combined organic phases were washed with saturated brine, dried over anhydrous sodium sulfate, filtered, and concentrated under reduced pressure to obtain brown oily intermediate 4-A (6.00 g, 95%), which was used directly in the next step without further purification.

Step 2: Synthesis of Intermediate 4-B

In nitrogen atmosphere at 0°C, a solution of bromine (9.88 g, 61.7 mmol, 2.00 eq.) in dichloromethane (30 mL) was added dropwise to a solution of intermediate 4-A (5.00 g, 30.9 mmol, 1.00 eq.) in dichloromethane (20 mL). The reaction mixture was stirred at room temperature overnight. The mixture was concentrated under reduced pressure to obtain brown oily intermediate 4-B (6.70 g, 90%), which was used directly in the next step without further purification.

Step 3: Synthesis of Compound 4

In nitrogen atmosphere at room temperature, Intermediate Int-1 (2.1 g, 10.3 mmol, 1.03 eq.) was added to a solution of intermediate 4-B (2.41 g, 10.0 mmol, 1.00 eq.) in tetrahydrofuran (20 mL). The reaction mixture was stirred at room temperature overnight. The mixture was poured into ice water (100 mL) and extracted with ethyl acetate (50 mL × 3). The combined organic phases were washed with saturated brine, dried over anhydrous sodium sulfate, filtered, and concentrated to dryness under reduced pressure. The crude product was purified by silica gel column chromatography (methanol: dichloromethane = 0% to 30%) to obtain Compound 4 (a yellow solid, 2.3 g, a yield of 67%).

LCMS (ESI): 347$[M+H]^+$.

TABLE 5 PREPARATION OF COMPOUNDS 4-2 TO 4-5 BY THE SYNTHETIC METHOD FOR COMPOUND 4 IN EXAMPLE 4

| Compound | Starting Material | Structural Formula | LCMS (ESI):$[M+H]^+$ |
|---|---|---|---|
| 4-2 | Int-2 | 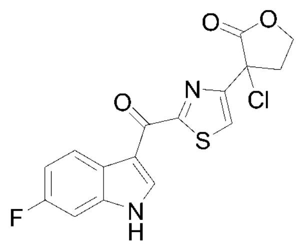 | LCMS (ESI): 365$[M+H]^+$ |
| 4-3 | Int-3 | 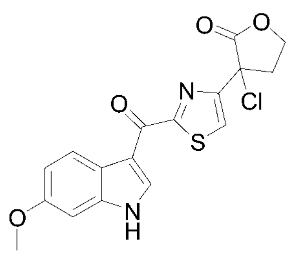 | LCMS (ESI): 377$[M+H]^+$ |
| 4-4 | Int-4 | 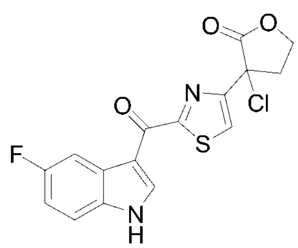 | LCMS (ESI): 365$[M+H]^+$ |

(continued)

| Compound | Starting Material | Structural Formula | LCMS (ESI):[M+H]$^+$ |
|---|---|---|---|
| 4-5 | Int-5 | 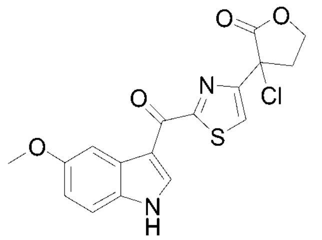 | LCMS (ESI): 377[M+H]$^+$ |

**EXAMPLE 5 SYNTHESIS OF COMPOUND 5**

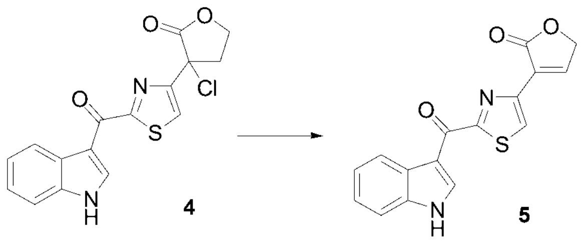

4 5

In nitrogen atmosphere at room temperature, triethylamine (58.5 mg, 0.578 mmol, 2.00 eq.) and triethylamine trihydrofluoride (93.2 mg, 0.578 mmol, 2.00 eq.) were added to a solution of compound 4 (0.100 g, 0.289 mmol, 1.00 eq.) in acetonitrile (3 mL). The mixture was stirred under reflux overnight. After cooling to room temperature, the mixture was concentrated to dryness under reduced pressure and purified by silica gel column chromatography (methanol: dichloromethane = 0% to 30%) to obtain Compound 5 (a yellow solid, 0.01 g, 11%).

LCMS (ESI): [M+H]$^+$ = 311.2. $^1$H NMR (400 MHz, DMSO-$d_6$) δ 12.30 (s, 1H), 9.27 (s, 1H), 8.51 (s, 2H), 8.33 (dd, *J* = 5.8, 3.2 Hz, 1H), 7.59 (dd, *J* = 5.8, 2.8 Hz, 1H), 7.37-7.16 (m, 2H), 5.17 (d, *J* = 1.3 Hz, 2H).

**EXAMPLE 6**

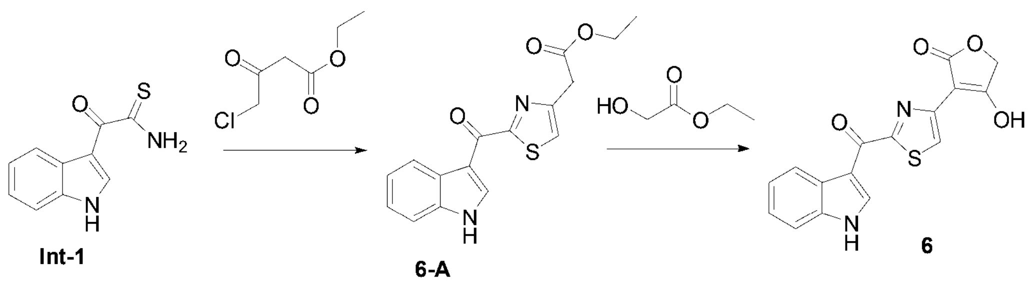

Int-1 6-A 6

Step 1: Synthesis of Intermediate 6-A

Intermediate 6-A was prepared by the same procedure as Step 2 for the synthesis of Compound 1, and the yield was 78%.

Step 2: Synthesis of Compound 6

In nitrogen atmosphere at room temperature, ethyl glycolate (0.13 g, 1.27 mmol, 4.00 eq.) and potassium tert-butoxide (0.29 g, 2.55 mmol, 8.00 eq.) were added to a solution of intermediate 6-A (0.10 g, 0.32 mmol, 1.00 eq.) in tetrahydrofuran (5 mL). The mixture was stirred at room temperature overnight. The mixture was poured into ice water (10 mL) and the pH was adjusted to 7 with a saturated aqueous tartaric acid solution. The mixture was extracted with ethyl

acetate (10 mL × 3). The combined organic phases were washed with saturated brine, dried over anhydrous sodium sulfate, filtered, and concentrated to dryness under reduced pressure. The crude product was purified by preparative high-performance liquid chromatography (HPLC) to obtain Compound 6 (a yellow solid, 45.00 mg, 43%).

LCMS $[M+H]^+$ = 327.0.

$^1H$ NMR (400 MHz, MeOH-*d4*) δ 9.50 (s, 1H), 8.39-8.37 (m, 1H), 7.96 (s, 1H), 7.53-7.44 (m, 1H), 7.30-7.19 (m, 2H), 4.54 (s, 2H).

**EXAMPLE 7**

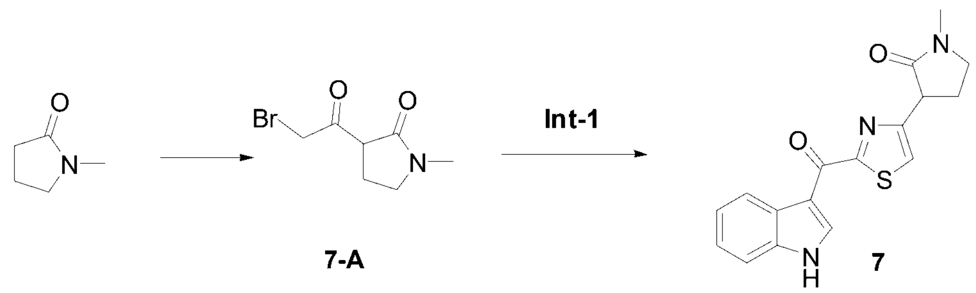


7-A

7

Step 1: Synthesis of Intermediate 7-A

A freshly prepared solution of lithium diisopropylamide (LDA), which was prepare by adding dropwise n-BuLi (4.04 mL, 10.09 mmol, 2.5 M in tetrahydrofuran (THF)) to a solution of diisopropylamine (1.42 mL, 10.09 mmol) in anhydrous THF (20 mL) at -78°C, was added dropwise to a solution of N-methyl-2-pyrrolidinone (1.0 g, 10.09 mmol) in anhydrous THF (15.0 mL) at -78°C. The mixture was stirred at the same temperature for 1 hour. A solution of ethyl bromoacetate (1.34 mL, 12.11 mmol) was added dropwise at -78°C over 5 minutes. The mixture was stirred at the same temperature for 3 hours. The temperature was slowly raised to -20°C. 1 N HCl (20 mL) was added dropwise while controlling the temperature not to exceed -20°C. The mixture was stirred for 10 to 15 minutes. The reaction mixture was diluted with ice water (30-40 g), and the layers were separated. The aqueous layer was extracted with ethyl acetate (EtOAc) (3 × 30 mL). The combined ethyl acetate phases were dried over $Na_2SO_4$ and concentrated under reduced pressure. The resulting crude residue was purified by column chromatography to obtain compound 7-A (a pale yellow oil, 1.230 g, a yield of 69%).

LCMS $[M+H]^+$ = 220, 222.

Step 2: Synthesis of Compound 7

The Compound 7 was prepared by the same procedure as Step 2 for the Synthesis of Compound 1. Intermediate 7-A (330 mg, 1.5 mmol) was added to a solution of intermediate Int-1 (300 mg, 1.46 mmol, 1.20 eq.) in tetrahydrofuran (10 mL) at room temperature. The mixture was stirred at room temperature in nitrogen atmosphere overnight. The mixture was concentrated to dryness under reduced pressure. Ice water was added, followed by stirring, and a solid was precipitated. The solid was filtered to obtain a crude product (152 mg). The crude product was recrystallized from ethyl acetate to obtain Compound 7 (a yellow solid, 120 mg, a yield of 54%).

LCMS (ESI): $[M+H]^+$ = 326.1.

$^1H$ NMR (400 MHz, DMSO-$d_6$) δ 12.25 (s, 1H), 8.99 (d, *J* = 3.2 Hz, 1H), 8.36 - 8.25 (m, 1H), 7.90 (s, 1H), 7.66 - 7.49 (m, 1H), 7.38 - 7.16 (m, 2H), 3.99 (t, *J* = 8.7 Hz, 1H), 3.61 - 3.41 (m, 2H), 2.84 (s, 3H), 2.55 - 2.49 (m, 1H), 2.38-2.33 (m, 1H).

TABLE 6 PREPARATION OF COMPOUNDS 7-2 TO 7-6 BY THE SYNTHETIC METHOD FOR COMPOUND 7 IN EXAMPLE 7

| Compound | Starting Material A | Starting Material B | Structural Formula | LCMS and NMR |
|---|---|---|---|---|
| 7-2 | 7-A | Int-2 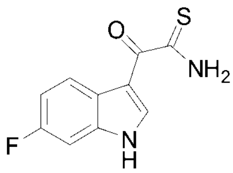 | 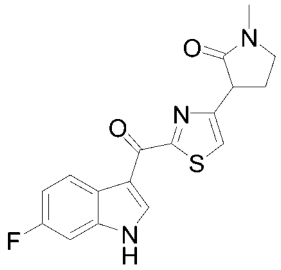 | LCMS(ESI):344.1[M+H]$^+$ $^1$H NMR (400 MHz, DMSO-$d_6$) δ 12.27 (s, 1H), 8.98 (s, 1H), 8.29 (dd, $J$ = 8.8, 5.6 Hz, 1H), 7.92 (s, 1H), 7.38 (dd, $J$ = 9.6, 2.3 Hz, 1H), 7.14 (td, $J$= 9.6, 2.4 Hz, 1H), 3.99 (t, $J$ = 8.7 Hz, 1H), 3.64 - 3.40 (m, 2H), 2.84 (s, 3H), 2.60 - 2.49 (m, 1H), 2.38-2.33 (m, 1H). |
| 7-3 | 7-A | Int-3 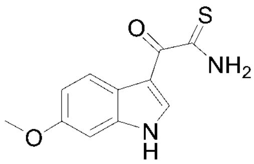 | 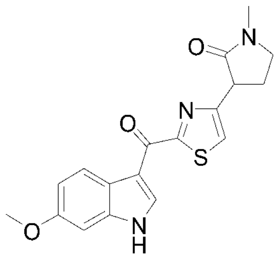 | LCMS(ESI):356.1[M+H]$^+$ $^1$H NMR (400 MHz, DMSO-$d_6$) δ 12.04 (s, 1H), 8.86 (s, 1H), 8.16 (d, $J$ = 8.7 Hz, 1H), 7.88 (s, 1H), 7.06 (d, $J$ = 2.2 Hz, 1H), 6.91 (dd, $J$ = 8.7, 2.3 Hz, 1H), 3.97 (t, $J$ = 8.7 Hz, 1H), 3.81 (s, 3H), 3.61 - 3.37 (m, 2H), 2.83 (s, 3H), 2.56 - 2.49 (m, 1H), 2.47-2.33 (m, 1H). |
| 7-4 | 7-A | Int-4 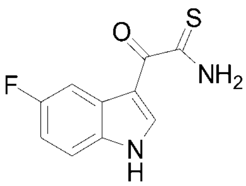 | 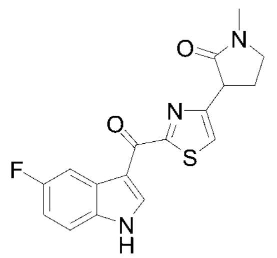 | LCMS(ESI):344. 1[M+H]$^+$ $^1$H NMR (400 MHz, DMSO-$d_6$) δ 12.35 (s, 1H), 9.02 (d, $J$ = 3.3 Hz, 1H), 7.98 (dd, $J$ = 9.9, 2.6 Hz, 1H), 7.92 (s, 1H), 7.59 (dd, $J$ = 8.8, 4.6 Hz, 1H), 7.15 (td, $J$ = 9.2, 2.6 Hz, 1H), 3.99 (t, $J$ = 8.7 Hz, 1H), 3.62 - 3.41 (m, 2H), 2.83 (s, 3H), 2.58 - 2.49 (m, 1H), 2.38-2.33 (m, 1H). |
| 7-5 | 7-A | Int-5 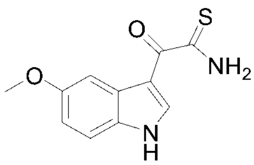 | 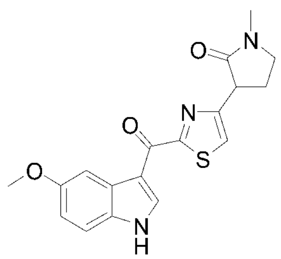 | LCMS(ESI):356.1[M+H]$^+$ $^1$H NMR (400 MHz, DMSO-$d_6$) δ 12.15 (d, $J$ = 2.4 Hz, 1H), 8.93 (d, $J$ = 3.3 Hz, 1H), 7.89 (s, 1H), 7.84 (d, $J$ = 2.5 Hz, 1H), 7.46 (d, $J$ = 8.8 Hz, 1H), 6.91 (dd, $J$ = 8.8, 2.6 Hz, 1H), 3.98 (t, $J$ = 8.7 Hz, 1H), 3.82 (s, 3H), 3.56 - 3.38 (m, 2H), 2.84 (s, 3H), 2.59 - 2.47 (m, 1H), 2.40-2.33 (m, 1H). |
| 7-6 | 7-A | Int-6 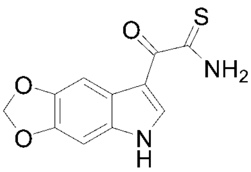 | 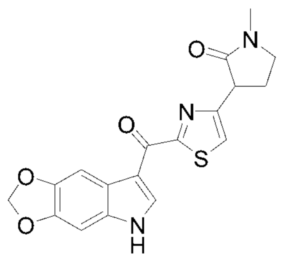 | LCMS(ESI):370. 1[M+H]$^+$ $^1$H NMR (400 MHz, DMSO-$d_6$) δ 12.05 (s, 1H), 8.79 (s, 1H), 7.88 (s, 1H), 7.70 (s, 1H), 7.10 (s, 1H), 6.04 (s, 2H), 3.97 (t, $J$ = 8.7 Hz, 1H), 3.62 - 3.40 (m, 2H), 2.83 (s, 3H), 2.58 - 2.47 (m, 1H), 2.36-2.31 (m, 1H). |

**EXAMPLE 8**

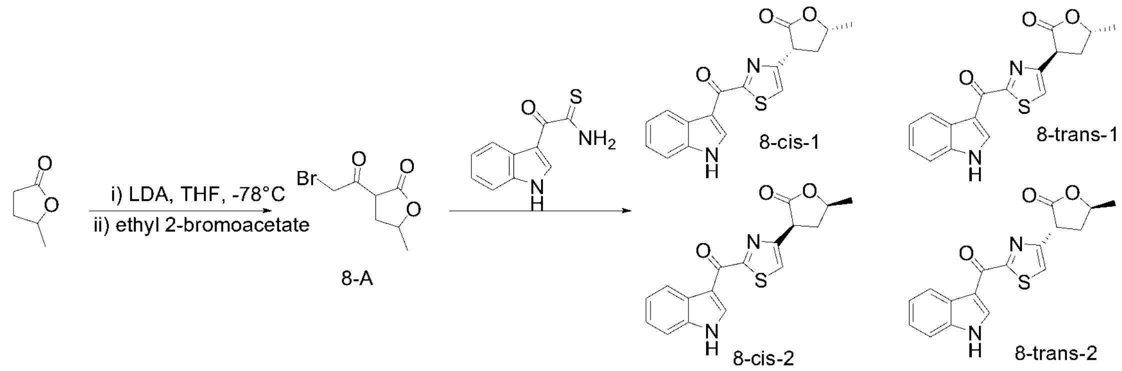


8-A

8-cis-1

8-trans-1

8-cis-2

8-trans-2

Step 1: Synthesis of Intermediate 8-A

A freshly prepared solution of LDA, which was prepared by adding dropwise n-BuLi (4.04 mL, 10.09 mmol, 2.5 M in THF to a solution of diisopropylamine (1.42 mL, 10.09 mmol) in anhydrous THF (20 mL) at -78°C, was slowly added dropwise to a solution of γ-valerolactone (1.1 g, 11 mmol) in anhydrous THF (15.0 mL) at -78°C. The mixture was stirred at the same temperature for 1 hour. At -78°C, a solution of ethyl bromoacetate (1.34 mL, 12.11 mmol) was added dropwise over 5 minutes. The mixture was maintained at the same temperature for 3 hours. The mixture was slowly warmed to -20°C. 1 N hydrochloric acid (HCl) (20 mL) was added dropwise while controlling the temperature not to exceed -20°C. The mixture was stirred for 10 to 15 minutes. The reaction mixture was diluted with ice water (30 g-40 g) and the layers were separated. The aqueous layer was extracted with EtOAc (3 × 30 mL). The combined ethyl acetate phases were dried over sodium sulfate ($Na_2SO_4$) and concentrated under reduced pressure. The resulting crude residue was purified by column chromatography to obtain compound 8-A (a pale yellow oil, 1.230 g, a yield of 69%).

LCMS $[M+H]^+$ = 221, 223.

Step 2: Synthesis of Compound 8

The Compound 8 was prepared by the same procedure as Step 2 for synthesis of compound 1. At room temperature, intermediate 8-A (332 mg, 1.5 mmol) was added to a solution of intermediate Int-1 (300 mg, 1.46 mmol, 1.20 eq.) in THF (10 mL). The mixture was stirred overnight at room temperature in nitrogen atmosphere. The mixture was concentrated to dryness under reduced pressure. Ice water was added and the mixture was stirred, resulting in precipitation of a solid. The solid was filtered to obtain a crude product (152 mg). The crude product was recrystallized from ethyl acetate to obtain Compound 8 (a yellow solid, 343 mg, a yield of 72%).

LCMS (ESI): $[M+H]^+$ = 327.1.

$^1H$ NMR (400 MHz, DMSO-$d_6$) δ 12.29 (s, 1H), 9.00 (d, *J* = 3.2 Hz, 0.6H), 8.99 (d, *J* = 3.2 Hz, 0.4H), 8.31 - 8.33 (m, 1H), 8.02(s, 0.6H), 8.01 (s, 0.4H), 7.55 - 7.58 (m, 1H), 7.25 - 7.32 (m, 2H), 4.95-5.00 (m, 0.4H), 4.72-4.77 (m, 0.6H), 4.48-4.54 (m, 1H), 2.83-2.90 (m, 0.6H), 2.72-2.79 (m, 0.4H), 2.32 - 2.44 (m, 0.4H), 2.24-2.32 (m, 0.6H), 1.48 (d, *J* = 8.0 Hz, 1.8H), 1.44 (d, *J* =8.0 Hz, 1.2H).

Step 3: Isomer Resolution

Compound 8 obtained from Step 2 was subjected to chromatographic separation. The resolution conditions were as follows:

Mobile Phase (Co_Solvent): MeOH [0.2% $NH_3$ (7M in MeOH)]
Column (Column Name): AD-3 4.6*100mm 3 um
Back Pressure: 2000 psi
Flow rate: 3.0 mL/min
Detector (Channel Name): PDA Ch1 MaxPlot (210-400 nm)
Detection Wavelength (Proc. Chnl. Descr.): PDA Ch1 MaxPlot (210-400 nm)
Column Temperature: 40°C
Run Time: 6.0 minutes

Four diastereomers were obtained after separation, which were 8-cis-1, 8-cis-2, 8-trans-1, and 8-trans-2.

TABLE 7 PREPARATION OF COMPOUNDS 8-2 TO 8-6 BY THE SYNTHETIC METHOD FOR COMPOUND 8 IN EXAMPLE 8

| Compound | Starting Material A | Starting Material B | Structural Formula | LCMS and NMR |
|---|---|---|---|---|
| 8-2 | 8-A | Int-2 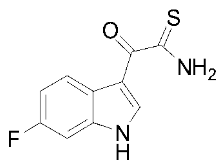 | 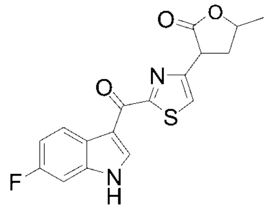 | LCMS(ESI):345.1$[M+H]^+$ $^1$H NMR (400 MHz, DMSO-$d_6$) $\delta$ 12.30 (s, 1H), 8.97+8.98 (two s, 1H), 8.30+8.28 (two d, $J$ = 8.0 Hz, 1H), 8.04+8.02 (two s, 1H), 7.40+7.38 (two d, 1H), 7.12-7.17 (m, 1H), 4.7-4.97 (m, 0.55H), 4.72-4.76 (m, 0.45H), -7.58 (m, 1H), 7.25 - 7.32 (m, 2H), 4.95-5.00 (m, 0.4H), 4.72-4.77 (m, 0.6H), 4.48-4.54 (m, 1H), 2.83-2.90 (m, 0.6H), 2.72-2.79 (m, 0.4H), 2.32 - 2.44 (m, 0.4H), 2.24-2.32 (m, 0.6H), 1.48 (d, $J$ = 8.0 Hz, 1.65H), 1.44 (d, $J$ =8.0 Hz, 1.35H) |
| 8-3 | 8-A | Int-3 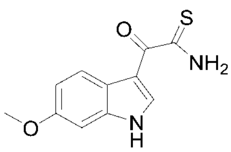 | 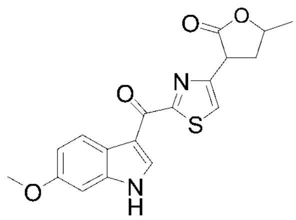 | LCMS(ESI):357.1$[M+H]^+$ $^1$H NMR (400 MHz, DMSO-$d_6$) $\delta$ 12.29 (s, 1H), 8.88+8.86 (two d, 1H), 8.16 (d, $J$ = 8.0 Hz, 1H), 8.01+7.99 (two s, 1H), 7.05+7.06 (two s, 1H), 6.90-6.92 (two d, 1H), 4.95-5.01 (m, 0.45H), 4.72-4.78 (m, 0.55H), 4.45-4.3(m, 1H), 3.81 (s, 3H), 2.83-2.90 (m, 0.55H), 2.70-2.78 (m, 0.45H), 2.35 - 2.44 (m, 0.45H), 2.22-2.31 (m, 0.55H), 1.48 (d, $J$ = 8.0 Hz, 1.65H), 1.44(d, $J$ =8.0 Hz, 1.35H) |
| 8-4 | 8-A | Int-4 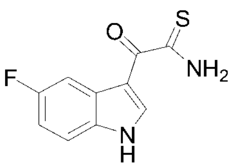 | 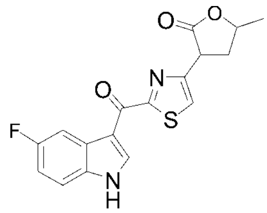 | LCMS(ESI):345.1$[M+H]^+$ $^1$H NMR (400 MHz, DMSO-$d_6$) $\delta$ 12.38 (s, 1H), 9.02+9.04 (two s, 1H), 8.04-7.97 (m, 2H), 7.98+7.99 (two s, 1H), 7.13-7.18 (m, 1H) , 4.95-5.00 (m, 0.45H), 4.72-4.77 (m, 0.55H), 4.48-4.53 (m, 1H), 2.74-2.88 (two m, 1H), 2.31-2.47 (m, 0.45H), 2.19-2.31 (m, 0.55H), 1.48 (d, $J$ = 8.0 Hz, 1.65H), 1.44(d, $J$ =8.0 Hz, 1.35H) |
| 8-5 | 8-A | Int-5 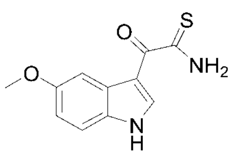 | 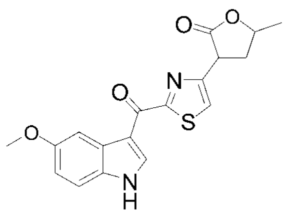 | LCMS(ESI):356.1$[M+H]^+$ $^1$H NMR (400 MHz, DMSO-$d_6$) $\delta$ 12.18 (s, 1H), 8.92+8.94 (two d, 1H), 8.01+7.99 (two s, 1H), 7.83+7.84 (two s, 1H), 7.45+7.47 (two s, 1H), 6.90-6.93 (two d, 1H), 4.93-5.01 (m, 0.45H), 4.71-4.75(m, 0.55H), 4.47-4.53(m, 1H), 3.82 (s, 3H), 2.83-2.90 (m, 0.55H), 2.72-2.78 (m, 0.45H), 2.37-2.44 (m, 0.45H), 2.22-2.31 (m, 0.55H), 1.48 (d, $J$ = 8.0 Hz, 1.65H), 1.44 (d, $J$ =8.0 Hz, 1.35H) |

(continued)

| Compound | Starting Material A | Starting Material B | Structural Formula | LCMS and NMR |
|---|---|---|---|---|
| 8-6 | 8-A | Int-6 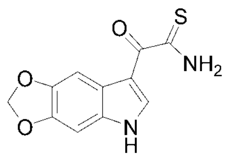 | 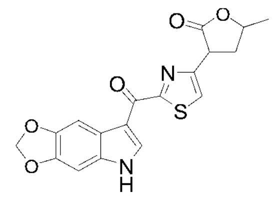 | LCMS(ESI):371.1[M+H]$^+$ $^1$H NMR (400 MHz, DMSO-$d_6$) δ 12.18 (s, 1H), 8.81+8.79 (two s, 1H), 8.00+7.98 (two s, 1H), 7.70 (two s, 1H) , 7.10 (s, 1H), 6.04 (s, 2H), 4.96-4.97 (m, 0.45H), 4.71-4.75 (m, 0.55H), 4.47-4.53 (m, 1H), 2.85-2.88 (m, 0.55H), 2.72-2.75 (m, 0.45H), 2.37-2.44 (m, 0.45H), 2.22-2.31 (m, 0.55H), 1.47 (d, *J* = 8.0 Hz, 1.65H), 1.43(d, *J* =8.0 Hz, 1.35H) |

**EXAMPLE 9**

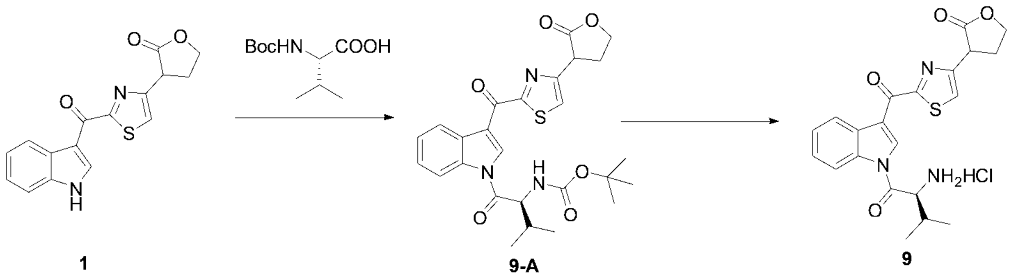

1 9-A 9

Step 1: Synthesis of Intermediate 9-A

3-[2-(1H-indole-3-carbonyl)-1,3-thiazol-4-yl]tetrahydrofuran-2-one (0.8 g, 2.56 mmol) was dissolved in N,N-dimethylformamide (15 mL). N-tert-butoxycarbonyl-L-valine (1.7 g, 7.825 mmol), O-(7-azabenzotriazol-1-yl)-N,N,N,N-tetramethyluronium hexafluorophosphate (2 g, 5.26 mmol), and N,N-diisopropylethylamine (4.7 mL, 27.2 mmol) were added sequentially. The reaction mixture was stirred at room temperature for 18 hours. The reaction was monitored by liquid chromatography-mass spectrometry (LC-MS) until completion. The reaction mixture was diluted with water and extracted with ethyl acetate. The organic phase was dried and concentrated under reduced pressure, followed by purification by silica gel column chromatography to obtain the target product tert-butyl {[(2S)-3-methyl-1-oxo-1-(3-{[4-(2-oxotetrahydrofuran-3-yl)-1,3-thiazol-2-yl]carbonyl}indol-1-yl)butan-2-yl]amino}carboxylate (Intermediate 9-A) (an off-white solid, 1.26 g, 2.46 mmol, 96.18%).

LCMS (ESI): [M+H]$^+$= 512.2.

$^1$H NMR (400 MHz, DMSO-$d_6$): δ 9.70-9.50 (m, 1H), 8.50-8.34 (m, 2H), 8.18-8.16 (m, 1H), 7.80-7.70 (m, 1H), 7.51-7.48 (m, 2H), 4.82-4.77 (m, 1H), 4.57-4.47 (m, 1H), 4.47-4.34 (m, 2H), 2.82-2.73 (m, 2H), 2.28-2.15 (m, 1H), 1.04-0.98 (m, 3H), 0.97-0.88 (m, 3H).

Step 2: Synthesis of Compound 9

Tert-butyl {[(2S)-3-methyl-1-oxo-1-(3-{[4-(2-oxotetrahydrofuran-3-yl)-1,3-thiazol-2-yl]carbonyl}indol-1-yl)butan-2-yl]amino}carboxylate (Intermediate 9-A) (0.1 g, 0.195 mmol) was dissolved in ethyl acetate (1.5 mL). A 4 N HCl/ethyl acetate solution (0.5 mL, 2 mmol) was added dropwise under an ice bath. The reaction mixture was stirred at room temperature for 18 hours. The reaction mixture was concentrated under reduced pressure. The residue was treated with n-hexane/tetrahydrofuran to obtain the product 3-[2-({1-[(2S)-2-amino-3-methylbutanoyl]indol-3-yllcarbonyl)-1,3-thiazol-4-yl]tetrahydrofuran-2-one hydrochloride (Compound 9) (a white solid powder, 70 mg, 0.153 mmol, 78.3%).

LCMS (ESI): [M+H]$^+$= 412.2.

$^1$H NMR (400 MHz, DMSO-$d_6$): δ 9.45-9.35 (m, 1H), 8.88-8.66 (br, 3H), 8.50-8.30 (m, 2H), 8.26-8.14 (m, 1H), 7.62-7.46 (m, 2H), 5.05-4.8 6 (m, 1H), 4.59-4.49 (m, 1H), 4.49-4.37 (m, 2H), 2.82-2.63 (m, 2H), 2.45-2.32 (m, 1H), 1.13-0.97 (m, 6H).

**EXAMPLE 10**

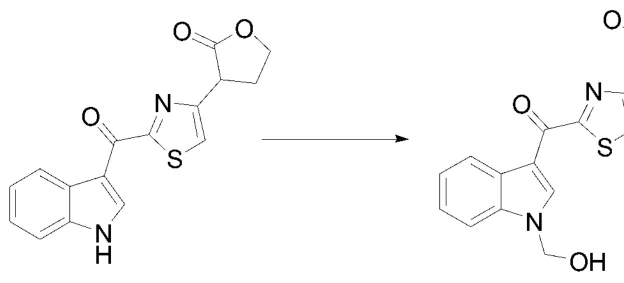

1 10

Step 1: Synthesis of Compound 10

3-[2-(1H-indole-3-carbonyl)-1,3-thiazol-4-yl]tetrahydrofuran-2-one (0.5 g, 1.6 mmol) was dissolved in tetrahydrofuran (5 mL). A 37% formaldehyde aqueous solution (5 mL) and tetrabutylammonium fluoride (42 mg, 0.16 mmol) were added. The reaction mixture was stirred at room temperature for 12 hours. The reaction was monitored by LC-MS until completion. The reaction mixture was diluted with ethyl acetate, washed with saturated brine, dried, and concentrated under reduced pressure. The residue was purified by silica gel column chromatography to obtain the product 3-{2-[1-(hydroxymethyl)indole-3-carbonyl]thiazol-4-yl}tetrahydrofuran-2-one (Compound 10) (a white solid, 420 mg, 1.17 mmol, 73.1%).
LCMS (ESI): $[M+H]^+$= 343.2.
$^1$H NMR (400 MHz, DMSO-$d_6$): δ 9.07 (s, 1H), 8.35-8.33 (m, 1H), 8.05 (s, 1H), 7.74-7.72 (m, 1H), 7.34-7.32 (m, 2H), 6.92-6.88 (t, *J*=7.6Hz, 1H), 5.70-5.68 (d, *J*=7.6Hz, 2H), 4.60-4.55 (m, 1H), 4.46-4.37 (m, 2H), 2.77-2.72 (m, 2H)

**EXAMPLE 11**

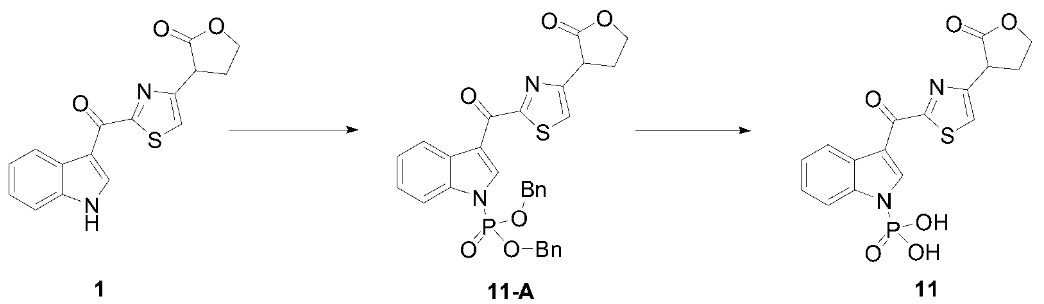

1 11-A 11

Step 1: Synthesis of Intermediate 11-A

3-[2-(1H-indole-3-carbonyl)-1,3-thiazol-4-yl]tetrahydrofuran-2-one (0.5 g, 1.6 mmol) was dissolved in anhydrous tetrahydrofuran (10 mL). A 0.5 M potassium bis(trimethylsilyl)amide toluene solution (3.2 mL) was added dropwise under an ice bath. After stirring for 20 minutes, a 0.4 M dibenzyl phosphorochloridate toluene solution (5 mL) was added dropwise slowly. The reaction mixture was stirred for 2 hours, and the reaction was monitored by LC-MS until completion. Saturated ammonium chloride (2 mL) was added to quench the reaction. The mixture was extracted with ethyl acetate. The organic phase was dried and concentrated under reduced pressure to obtain the product dibenzyl {3-[4-(2-oxotetrahydrofuran-3-yl)thiazole-2-carbonyl]-1H-indol-1-yl} phosphate (crude product 11-A) (a brown solid, 0.92 g, 1.6 mmol).
LCMS (ESI): $[M+H]^+$= 573.4.

Step 2: Synthesis of Compound 11

Dibenzyl {3-[4-(2-oxotetrahydrofuran-3-yl)thiazole-2-carbonyl]-1H-indol-1-yl} phosphate (0.92 g, 1.6 mmol) was dissolved in methanol (40 mL). 20% Palladium hydroxide on carbon (1.12 g, 1.6 mmol) was added. After hydrogen displacement, the hydrogenation reaction was carried out for 8 hours. The reaction was monitored by LC-MS until completion. The reaction mixture was filtered and concentrated under reduced pressure. The residue was purified by reverse-phase column chromatography to obtain the product {3-[4-(2-oxotetrahydrofuran-3-yl)thiazole-2-carbonyl]-1H-indol-1-yl} phosphoric acid (Compound 11) (a yellow solid, 0.38 g, 0.97 mmol, 60.6%).
LCMS (ESI): $[M+H]^+$= 393.4.
$^1H$ NMR (400 MHz, DMSO-$d_6$): δ 9.03-9.01 (m, 1H), 8.66 (s, 1H), 8.28-8.20 (m, 1H), 8.01-7.96 (m, 1H), 7.36-7.26 (m, 2H), 4.58-4.52 (m, 1H), 4.50-4.36 (m, 2H), 2.80-2.70 (m, 2H).

**EXAMPLE 12**

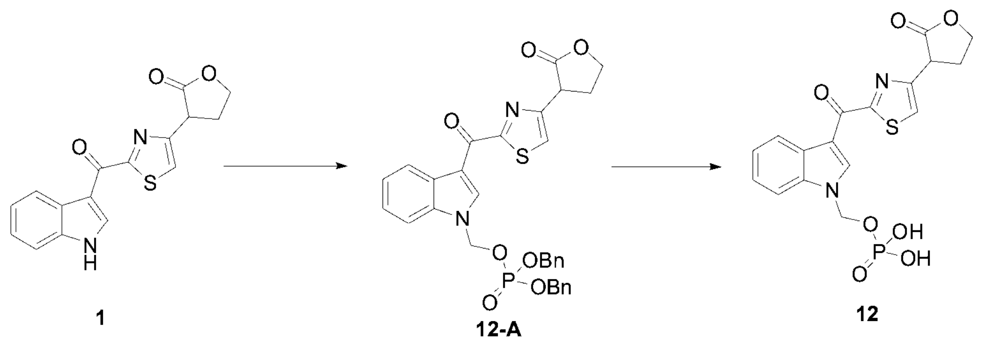

1 12-A 12

Step 1: Synthesis of Intermediate 12-A

3-[2-(1H-indole-3-carbonyl)-1,3-thiazol-4-yl]tetrahydrofuran-2-one (0.5 g, 1.6 mmol) was dissolved in anhydrous tetrahydrofuran (10 mL). A 0.5 M potassium bis(trimethylsilyl)amide toluene solution (3.2 mL) was added dropwise under an ice bath. After stirring for 20 minutes, dibenzyl chloromethylphosphonate (522 mg, 1.6 mmol) was added. The reaction mixture was stirred at room temperature overnight. The reaction was monitored by LC-MS until completion. The reaction mixture was concentrated under reduced pressure. The residue was purified by silica gel column chromatography to obtain the product dibenzyl {3-[4-(2-oxotetrahydrofuran-3-yl)thiazole-2-carbonyl]-1H-indol-1-yl}methylphosphate (Intermediate 12-A) (a yellow oil, 512 mg, 0.85 mmol).
LCMS (ESI): $[M+H]^+$= 603.4.

Step 2: Synthesis of Compound 12

Dibenzyl {3-[4-(2-oxotetrahydrofuran-3-yl)thiazole-2-carbonyl]-1H-indol-1-yl}methylphosphate (0.51 g, 0.85 mmol) was dissolved in methanol (20 mL). 20% Palladium hydroxide on carbon (0.6 g, 0.85 mmol) was added. After hydrogen displacement, the hydrogenation reaction was carried out for 8 hours. The reaction was monitored by LC-MS until completion. The reaction mixture was filtered and concentrated under reduced pressure. The residue was purified by reverse-phase column chromatography to obtain the product {3-[4-(2-oxotetrahydrofuran-3-yl)thiazole-2-carbonyl]-1H-indol-1-yl}methylphosphoric acid (Compound 12) (a yellow solid, 64 mg, 0.15 mmol, 17.6%).
LCMS (ESI): $[M+H]^+$= 423.4.
$^1H$ NMR (400 MHz, DMSO-$d_6$): δ 9.05-9.01 (m, 1H), 8.67-8.62 (m, 1H), 8.30-8.25 (m, 1H), 7.81-7.78 (m, 1H), 7.46-7.39 (m, 2H), 5.90-5.85 (m, 2H), 4.60-4.55 (m, 1H), 4.50-4.37 (m, 2H), 2.84-2.72 (m, 2H).

**EXAMPLE 13**

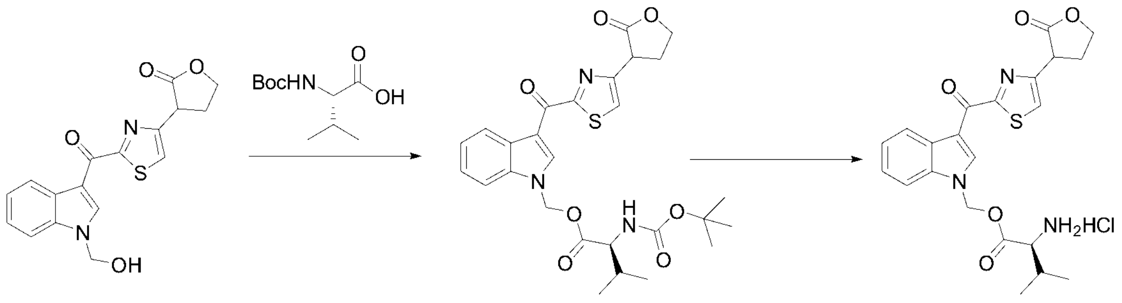

**10** **13-A** **13**

Step 1: Synthesis of Intermediate 13-A

3-{2-[1-(hydroxymethyl)indole-3-carbonyl]thiazol-4-yl}tetrahydrofuran-2-one (Compound 10) (0.44 g, 1.28 mmol) was dissolved in N,N-dimethylformamide (6 mL). N-tert-butoxycarbonyl-L-valine (0.42 g, 1.92 mmol), O-(7-azabenzotriazol-1-yl)-N,N,N,N-tetramethyluronium hexafluorophosphate (0.73 g, 1.92 mmol), and N,N-diisopropylethylamine (0.67 mL, 3.84 mmol) were added sequentially. After stirring at room temperature for 18 hours, the reaction was monitored by LC-MS until completion. The reaction mixture was diluted with ethyl acetate and washed with saturated brine. The organic phase was dried and concentrated under reduced pressure. The residue was purified by silica gel column chromatography to obtain the product {3-[4-(2-oxotetrahydrofuran-3-yl)thiazole-2-carbonyl]-1H-indol-1-yl} -N-tert-butoxycarbonyl-L-valine methyl ester (13-A) (a colorless oil, 206 mg, 0.38 mmol, 29.7%).

LCMS (ESI): $[M+H]^+= 542.2$.

Step 2: Synthesis of Compound 13

{3-[4-(2-oxotetrahydrofuran-3-yl)thiazole-2-carbonyl]-1H-indol-1-yl}-N-tert-butoxycarbonyl-L-valine methyl ester (13-A) (0.2 g, 0.37 mmol) was dissolved in ethyl acetate (3 mL). A 4N HCl/ethyl acetate solution (1 mL, 4 mmol) was added under an ice bath. The reaction mixture was stirred at room temperature for 18 hours. The reaction system was concentrated under reduced pressure. The residue was treated with n-hexane/tetrahydrofuran to obtain the product {3-[4-(2-oxotetrahydrofuran-3-yl)thiazole-2-carbonyl]-1H-indol-1-yl}-L-valine methyl ester hydrochloride (Compound 13) (a white solid powder, 72 mg, 0.15 mmol, 40.8%).

LCMS (ESI): $[M+H]^+= 442.2$.

$^1H$ NMR (400 MHz, DMSO-$d_6$): $\delta$ 9.20 (s, 1H), 8.82-8.68 (br, 3H), 8.40-8.38 (m, 1H), 8.13 (s, 1H), 7.81-7.76 (m, 1H), 7.45-7.40 (m, 2H), 6.60-6.42 (m, 2H), 5.02-4.96 (m, 1H), 4.74-4.64 (m, 1H), 4.53-4.42 (m, 2H), 2.85-2.82 (m, 2H), 2.12-1.98 (m, 1H), 1.13-0.97 (m, 6H).

**EXAMPLE 14**

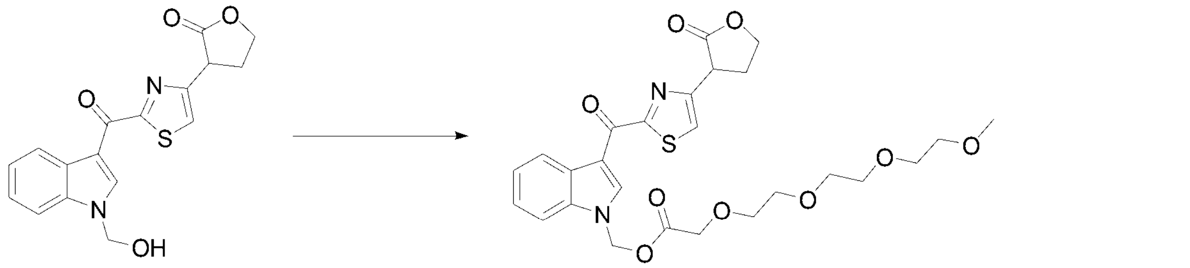

**10** **14**

Step 1: Synthesis of Compound 14

3-{2-[1-(hydroxymethyl)indole-3-carbonyl]thiazol-4-yl}tetrahydrofuran-2-one (Compound 10) (0.2 g, 0.58 mmol) was dissolved in N,N-dimethylformamide (5 mL). 3,6,9,12-tetraoxatridecanoic acid (129 mg, 0.58 mmol), O-(7-azabenzotriazol-1-yl)-N,N,N,N-tetramethyluronium hexafluorophosphate (0.33 g, 0.87 mmol), and N,N-diisopropylethylamine (0.2 mL, 1.16 mmol) were added sequentially. The reaction mixture was stirred at room temperature for 18 hours, and the

reaction was monitored by LC-MS until completion. The reaction system was diluted with water and extracted with ethyl acetate. The organic phase was dried and concentrated under reduced pressure. The residue was purified by silica gel column chromatography to obtain the product 3,6,9,12-tetraoxatridecanoic acid {3-[4-(2-oxotetrahydrofuran-3-yl)thiazole-2-carbonyl]-1H-indol-1-yl}methyl ester (Compound 14) (a colorless oil, 0.115 g, 0.21 mmol, 36.2%).

LCMS (ESI): $[M+H]^+$= 547.2.

$^1$H NMR (400 MHz, DMSO-$d_6$): δ 9.10 (s, 1H), 8.45-8.40 (m, 1H), 8.32 (s, 1H), 7.70-7.60 (m, 1H), 7.40-7.35 (m, 2H), 6.28 (s, 2H), 4.55-4.50 (m, 1H), 4.48-4.38 (m, 2H), 4.20 (s, 2H), 3.72-3.65(m, 4H), 3.64-3.58 (m, 6H), 3.54-3.52 (m, 2H), 3.36 (s, 3H), 2.78-2.70 (m, 2H).

## EXAMPLE 15

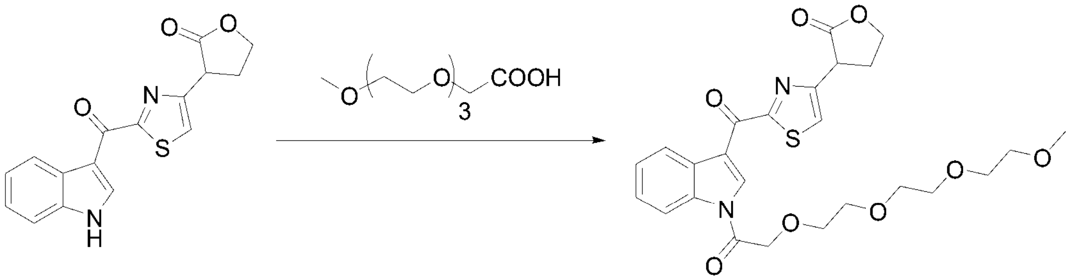


1 15

Step 1: Synthesis of Compound 15

3-[2-(1H-indole-3-carbonyl)-1,3-thiazol-4-yl]tetrahydrofuran-2-one (0.4 g, 1.28 mmol) was dissolved in N,N-dimethylformamide (6 mL). 3,6,9,12-tetraoxatridecanoic acid (568 mg, 2.56 mmol), O-(7-azabenzotriazol-1-yl)-N,N,N,N-tetramethyluronium hexafluorophosphate (0.97 g, 2.56 mmol), and N,N-diisopropylethylamine (1.34 mL, 7.68 mmol) were added sequentially. The reaction mixture was stirred at room temperature for 18 hours, and the reaction was monitored by LC-MS until completion. The reaction system was diluted with water and extracted with ethyl acetate. The organic phase was dried and concentrated under reduced pressure. The residue was purified by silica gel column chromatography to obtain the product 3-{2-[1-(3,6,9,12-tetraoxatridecanoyl)-1H-indole-3-carbonyl]thiazol-4-yl}dihydrofuran-2(3H)-one (Compound 15) (a colorless oil, 0.21 g, 0.4 mmol, 31.5%).

LCMS (ESI): $[M+H]^+$= 517.2.

$^1$H NMR (400 MHz, DMSO-$d_6$): δ 9.11 (s, 1H), 8.48-8.42 (m, 1H), 8.33 (s, 1H), 7.72-7.66 (m, 1H), 7.42-7.36 (m, 2H), 4.58-4.52 (m, 1H), 4.50-4.41 (m, 2H), 4.22 (s, 2H), 3.74-3.65(m, 4H), 3.64-3.56 (m, 6H), 3.54-3.52 (m, 2H), 3.38 (s, 3H), 2.77-2.72 (m, 2H).

## EFFICACY EXAMPLE 1: HUMAN AHR AGONIST LUCIFERASE ASSAY

Cytochrome P450 family 1 member A1 (CYP1A1) is one of the hallmark genes regulated by the AHR signaling pathway. The upstream promoter of the CYP1A1 gene includes a specific sequence called a dioxin responsive element (DRE), which binds to an activated AHR to initiate CYP1A1 expression. To detect AHR activation, a reporter gene plasmid was constructed by placing the gene sequence from -1200 bp to 0 bp upstream of CYP1A1 in front of a Nano-luc luciferase reporter gene according to conventional construction methods of reporter gene plasmids in the art. The reporter gene plasmid was transfected into human hepatoma HepG2 cells by a conventional liposome transfection method in the art, and stably expressing cells were selected by puromycin. The selected cells were diluted to a concentration of 5 cells/mL and seeded into a 96-well plate at 100 μL/well, and single clones were obtained after growth. A single clone with normal morphology, normal growth, and the highest induction signal was selected for the human AHR agonist luciferase assay.

On day one, the HepG2 monoclonal cell line stably expressing reporter gene was counted and seeded into a white opaque 96-well plate at a density of 1-8×$10^4$ cells per well using 100 μL of complete medium without tryptophan and phenol red (minimal essential medium (MEM) without tryptophan and phenol red: , 10% fetal bovine serum, 1× non-essential amino acids, 1× sodium pyruvate, and 1× GlutaMax). The cells were cultured in an incubator maintained at 37°C with 5% $CO_2$ for 24 hours.

Test compounds were prepared as 10 mM stock solutions in dimethyl sulfoxide (DMSO) and stored at 4°C protected from light. Before treatment, the compound stock solutions, positive control compounds (e.g., dioxin, 2-(1H-indole-3-carbonyl)thiazole-4-carboxylic acid methyl ester (ITE), or kynurenine), and a negative control compound (DMSO) were subjected to preliminary dilution (determined based on pre-experiments) with the complete medium without tryptophan and phenol red, followed by 3- to 20-fold serial dilutions to 11 concentration points to obtain 2× dilution solutions. When treating the cells, 50 μL of medium was first aspirated from the 96-well plate containing the seeded cells, and then 50 μL of the serially diluted 2× dilution solution was added. Each compound was tested in duplicate, and each duplicate experiment included a well with a concentration of 0 (i.e., containing only the medium) to determine the baseline. The reporter cells were returned to the incubator and incubated for 4 to 24 hours after treatment.

After treatment, 100 μL/well of Nano-Glo Luciferase Assay Reagent was added to the 96-well plate, and the relative light unit (RLU) of each well was measured. The RLU values for the wells with the concentration of 0 on the 96-well plates were averaged to calculate the baseline Ave $RLU^{Vehicle}$. The activity of AHR induced by the test compounds at different concentrations was calculated according to Formula (1) based on the ratio of $RLU^{Test\ Cmpd}$ from the experimental groups to the baseline Ave $RLU^{Vehicle}$, to determine the activation fold.

$$activation\ fold = \frac{RLU^{Test\ Cmpd}}{Ave\ RLU^{Vehicle}} \quad \text{Formula (1).}$$

The activation fold and the corresponding compound concentration were fitted using GraphPad Prism 9 with [Agonist] vs. response - variable slope (four parameters) to calculate the $EC_{50}$ of the compound for AHR activation. The $EC_{50}$ values for the compounds are shown in Table 8, where A indicates $EC_{50} \leq 500$ nM, B indicates 500 nM < $EC_{50} \leq 2.0$ μM, and C indicates 2.0 μM < $EC_{50} \leq 100$ μM.

**EFFICACY EXAMPLE 2: MOUSE AHR AGONIST LUCIFERASE ASSAY**

To detect AHR activation, a reporter gene plasmid was constructed by placing six tandem xenobiotic responsive elements (XREs, see Buckley, S. M. K. et al., Sci. Rep. 2015; 5: 11842.) in front of a Nano-luc luciferase reporter gene according to conventional construction methods of reporter gene plasmids in the art. The reporter gene plasmid was transfected into mouse hepatoma Hepa1-6 cells by a conventional liposome transfection method in the art, and stably expressing cells were selected by puromycin for the mouse AHR agonist luciferase assay.

On day one, the stably expressing reporter gene Hepa1-6 cell line was counted and seeded into a white opaque 96-well plate at a density of $1\text{-}8\times10^4$ cells per well using 100 μL of phenol red-free Dulbecco's Modified Eagle Medium (DMEM) (DMEM without phenol red, 10% fetal bovine serum). The cells were cultured in an incubator maintained at 37°C with 5% $CO_2$ for 24 hours.

Test compounds were prepared as 10 mM stock solutions in DMSO and stored at 4°C protected from light. Before treatment, the compound stock solutions, positive control compounds (e.g., dioxin, ITE, or kynurenine), and a negative control compound (DMSO) were subjected to preliminary dilution (determined based on pre-experiments) with complete medium without tryptophan and phenol red, followed by 3- to 20-fold serial dilutions to 11 concentration points to obtain 10× dilution solutions. When treating the cells, 10 μL of medium was first aspirated from the 96-well plate containing the seeded cells, and then 10 μL of the serially diluted 10× dilution solution was added. Each compound was tested in duplicate, and each duplicate experiment included a well with a concentration of 0 (i.e., containing only medium) to determine the baseline. The reporter cells were returned to the incubator and incubated for 4 to 24 hours after treatment.

After treatment, 100 μL/well of Nano-Glo Luciferase Assay Reagent was added to the 96-well plate, and the RLU of each well was measured. The RLU values for the wells with the concentration of 0 on the 96-well plates were averaged to calculate the baseline Ave $RLU^{Vehicle}$. The activity of AHR induced by the test compounds at different concentrations was calculated according to Formula (1) based on the ratio of $RLU^{Test\ Cmpd}$ from the experimental groups to the baseline Ave $RLU^{Vehicle}$, to determine the activation fold.

$$activation\ fold = \frac{RLU^{Test\ Cmpd}}{Ave\ RLU^{Vehicle}} \quad \text{Formula (1).}$$

The activation fold and the corresponding compound concentration were fitted using GraphPad Prism 9 with [Agonist] vs. response - variable slope (four parameters) to determine the $EC_{50}$ of the compound for AHR activation. The $EC_{50}$ values for the compounds are shown in Table 8, where A indicates $EC_{50} \leq 100$ nM, B indicates 100 nM < $EC_{50} \leq 2.0$ μM, and C indicates 2.0 μM < $EC_{50} \leq 100$ μM.

**EFFICACY EXAMPLE 3: RAT AHR AGONIST LUCIFERASE ASSAY**

To detect AHR activation, a Nano-luc luciferase reporter gene linked to six tandem xenobiotic responsive elements (XREs) (see Buckley, S. M. K. et al., Sci. Rep. 2015; 5: 11842.) was synthesized and cloned into a lentiviral

transfer plasmid pGWLV11-new using KpnI and XbaI restriction sites. A lentivirus containing the reporter gene was generated by co-transfecting the transfer plasmid with packaging plasmids into 293T cells using conventional transfection methods in the art, followed by purification. The reporter gene was introduced into a genome of rat hepatoma H-4-II-E cells via lentiviral infection, and cells stably expressing the reporter gene were selected with puromycin for the rat AHR agonist luciferase assay.

On day one, the H-4-II-E cell line stably expressing the reporter gene was counted and seeded into a white opaque 96-well plate at a density of 1-8×$10^4$ cells per well using 100 μL of complete medium without tryptophan and phenol red (MEM without tryptophan and phenol red, 10% fetal bovine serum, 1× non-essential amino acids, 1× sodium pyruvate, and 1× GlutaMax). The cells were cultured in an incubator maintained at 37°C with 5% $CO_2$ for 24 hours.

Test compounds were prepared as 10 mM stock solutions in DMSO and stored at 4°C protected from light. Before treatment, the compound stock solutions, positive control compounds (e.g., dioxin, ITE, or kynurenine), and a negative control compound (DMSO) were subjected to a preliminary dilution (determined based on pre-experiments) with the complete medium without tryptophan and phenol red, followed by 3- to 20-fold serial dilutions to 11 concentration points to obtain 2× dilution solutions. When treating the cells, 50 μL of medium was first aspirated from the 96-well plate containing the seeded cells, and then 50 μL of the serially diluted 2× dilution solutions was added. Each compound was tested in duplicate, and each duplicate experiment included a well with a concentration of 0 (i.e., containing only the medium) to determine the baseline. The reporter cells were returned to the incubator and incubated for 4 to 24 hours after treatment.

After treatment, 100 μL/well of Nano-Glo Luciferase Assay Reagent was added to the 96-well plates, and the RLU of each well was measured. The RLU values for the wells with the concentration of 0 on the 96-well plates were averaged to calculate the baseline Ave $RLU^{Vehicle}$. The activity of AHR induced by the test compounds at different concentrations was calculated according to Formula (1) based on the ratio of $RLU^{Test\ Cmpd}$ from the experimental groups to the baseline Ave $RLU^{Vehicle}$, to determine the activation fold.

$$activation\ fold = \frac{RLU^{Test\ Cmpd}}{Ave\ RLU^{Vehicle}} \quad \text{Formula (1).}$$

The activation fold and the corresponding compound concentration were fitted using GraphPad Prism 9 with [Agonist] vs. response - variable slope (four parameters) to calculate the $EC_{50}$ of the compound for AHR activation. The $EC_{50}$ values for the compounds are shown in Table 8, where A indicates $EC_{50} \leq 10$ nM, B indicates 10 nM < $EC_{50} \leq 2.0$ μM, and C indicates 2.0 μM < $EC_{50} \leq 100$ μM.

TABLE 8 $EC_{50}$ VALUES OF THE COMPOUNDS

| Compound No. | HepG2 $EC_{50}$ | Hepa 1-6 $EC_{50}$ | H4IIE $EC_{50}$ |
|---|---|---|---|
| 1 | A | A | A |
| 1-isomer-A | A | A | A |
| 1-isomer-B | A | A | A |
| 1-2 | A | A | A |
| 1-3 | A | A | A |
| 1-4 | A | A | A |
| 1-5 | A | A | A |
| 1-6 | A | A | A |
| 1-7 | A | - | - |
| 1-8 | A | - | - |
| 1-9 | A | - | - |
| 2 | A | A | A |
| 2-2 | A | A | A |
| 2-3 | A | A | A |
| 2-4 | A | A | - |
| 2-5 | A | A | - |
| 3 | A | A | - |
| 3-2 | A | A | - |
| 3-3 | A | A | - |
| 3-4 | A | B | - |
| 3-5 | B | - | - |

(continued)

| Compound No. | HepG2 $EC_{50}$ | Hepa 1-6 $EC_{50}$ | H4IIE $EC_{50}$ |
|---|---|---|---|
| 4 | A | A | A |
| 4-2 | A | A | A |
| 4-3 | A | - | - |
| 4-4 | A | - | - |
| 4-5 | A | - | - |
| 5 | A | A | - |
| 6 | B | - | - |
| 7 | A | - | C |
| 7-2 | A | - | C |
| 7-3 | A | - | C |
| 7-4 | A | - | C |
| 7-5 | A | A | C |
| 7-6 | A | A | C |
| 8 | A | A | - |
| 8-cis-1 | A | - | - |
| 8-cis-2 | A | - | - |
| 8-trans-1 | A | - | - |
| 8-trans-2 | A | - | - |
| 8-1 | A | A | - |
| 8-2 | A | A | - |
| 8-3 | A | A | - |
| 8-4 | A | A | - |
| 8-5 | A | A | - |
| 8-6 | A | A | - |
| 9 | B | B | - |
| 10 | B | B | - |
| 11 | B | B | - |
| 12 | B | B | - |
| 13 | B | B | - |
| 14 | B | B | - |
| 15 | B | B | - |
| ITE | A | B | B |

As shown in Table 8, the compounds of the present invention activate expression of CYP1A1 gene and exhibit a strong agonistic activity on AHR.

## Claims

**1.** An aryl hydrocarbon receptor modulator, or an enantiomer, a prodrug, or a pharmaceutically acceptable salt thereof, comprising the following general formula (I):

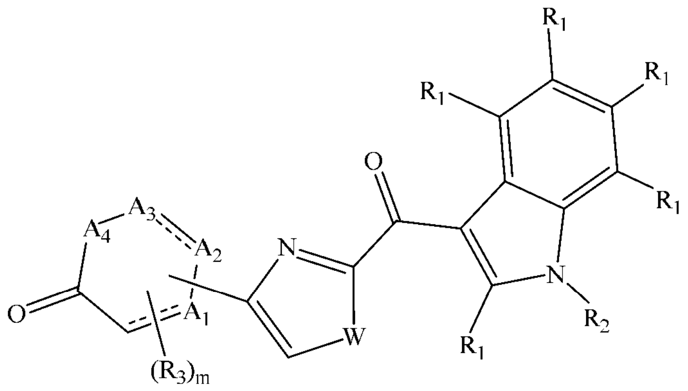

(I),

wherein W is C=C or S;
$A_1$, $A_2$, and $A_3$ are each independently a bond or C; m is an integer from 1 to 7; $R_3$ is independently H, D, halogen, CN, OR, SR, N(R)R, -C(O)R, -C(O)OR, RC(O)O-, $S(O)_nR$, unsubstituted C1-C8 alkyl or substituted C1-C8 alkyl with 1-6 $R_a$, unsubstituted C2-C8 alkenyl or substituted C2-C8 alkenyl with 1-6 $R_a$, unsubstituted C2-C8 alkynyl or substituted C2-C8 alkynyl with 1-6 $R_a$, or unsubstituted C3-C10 cycloalkyl or substituted C3-C10 cycloalkyl with 1-6 $R_a$; and $R_a$ is D, halogen, OR, SR, N(R)R, -C(O)R, -C(O)OR, RC(O)O-, or $S(O)_nR$;
$A_4$ is independently O or $NR_4$; $R_4$ is H, unsubstituted C1-C8 alkyl or substituted C1-C8 alkyl with 1-6 $R_b$, unsubstituted C2-C8 alkenyl or substituted C2-C8 alkenyl with 1-6 $R_b$, unsubstituted C2-C8 alkynyl or substituted C2-C8 alkynyl with 1-6 $R_b$, or unsubstituted C3-C10 cycloalkyl or substituted C3-C10 cycloalkyl with 1-6 $R_b$; and $R_b$ is D, halogen, OR, SR, N(R)R, -C(O)R, - C(O)OR, RC(O)O-, or $S(O)_nR$;
$R_1$ is independently H, D, halogen, CN, OR, SR, N(R)R, -C(O)R, -C(O)OR, RC(O)O-, $S(O)_nR$, unsubstituted C1-C8 alkyl or substituted C1-C8 alkyl with 1-6 $R_b$, unsubstituted C2-C8 alkenyl or substituted C2-C8 alkenyl with 1-6 $R_b$, unsubstituted C2-C8 alkynyl or substituted C2-C8 alkynyl with 1-6 $R_b$, or unsubstituted C3-C10 cycloalkyl or substituted C3-C10 cycloalkyl with 1-6 $R_b$; $R_b$ is D, halogen, OR, SR, N(R)R, -C(O)R, -C(O)OR, RC(O)O-, or $S(O)_nR$; or two adjacent or meta $R_1$ groups form a 3-10 membered carbocycle or a 3-10 membered heterocycle containing 1 to 3 heteroatoms selected from N, O, or S;
n is 1 or 2;
R is independently H, C1-C8 alkyl, C2-C8 alkenyl, C2-C8 alkynyl, or C3-C10 cycloalkyl; and
$R_2$ is H, $-(CH_2O)_aC(O)(CH_2)_b(O)_c(CH(R_c))_d(CH_2CH_2O)_eR_c$, $-(CH_2O)_aP(O)(R_p)_2$, or - $(CH_2O)_a(CH(R_c))_d(CH_2CH_2O)_eR_c$, $R_p$ is independently selected form $(CH_2)_b(O)_c(CH(R_c))_d(CH_2CH_2O)_eR_c$; $R_c$ is independently H, D, C1-C8 alkyl, $NH_2$-, (C1-C8 alkyl)NH-, $(C1\text{-}C8\ alkyl)_2N$-, $NH_2$(C1-C8 alkyl)-, OH, HO-(C1-C8 alkyl)-, or (C1-C8 alkyl)O-; a, b, c, and d are each independently 0 or 1; and e is an integer from 0 to 3.

2. The aryl hydrocarbon receptor modulator according to claim 1, wherein when W is S, the general formula (I) is a general formula (Ia):

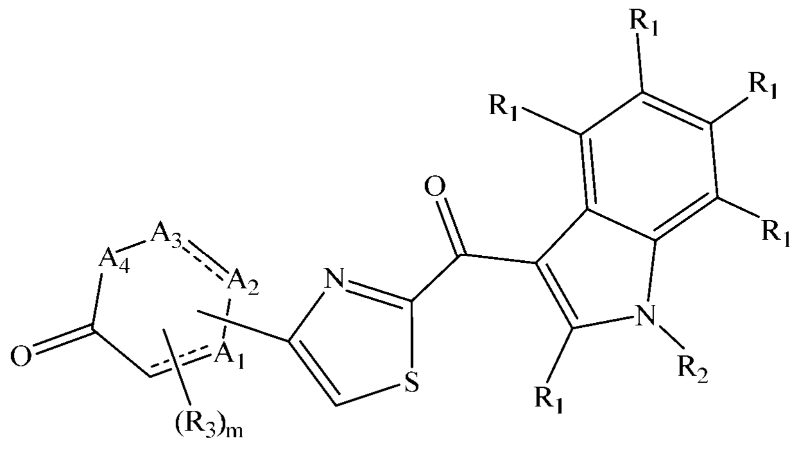

(Ia).

3. The aryl hydrocarbon receptor modulator according to claim 2, wherein when $A_4$ is O, the general formula (Ia) is a general formula (Ia1):

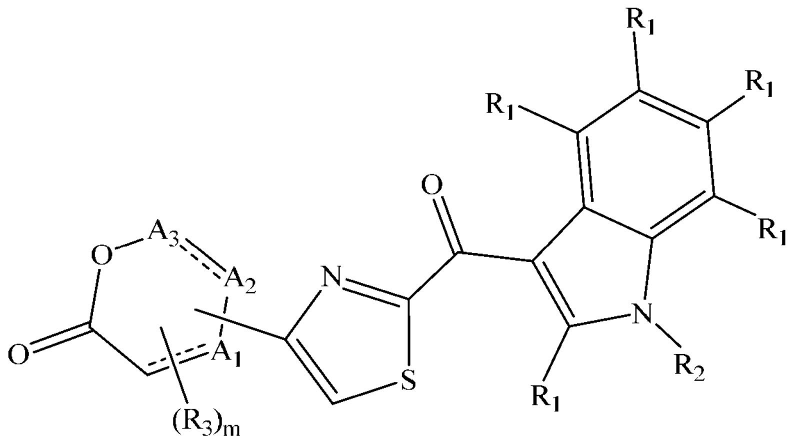

(Ia1);

or when $A_4$ is $NR_4$, the general formula (Ia) is a general formula (Ia2):

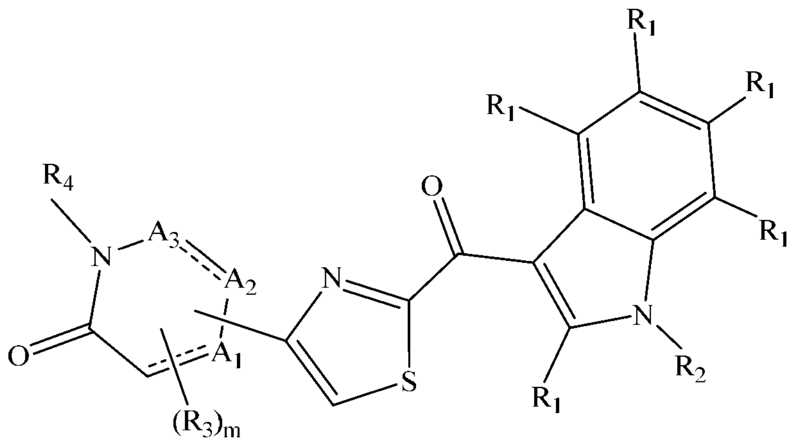

(Ia2).

**4.** The aryl hydrocarbon receptor modulator according to claim 1, wherein when W is C=C, the general formula (I) is a general formula (Ib):

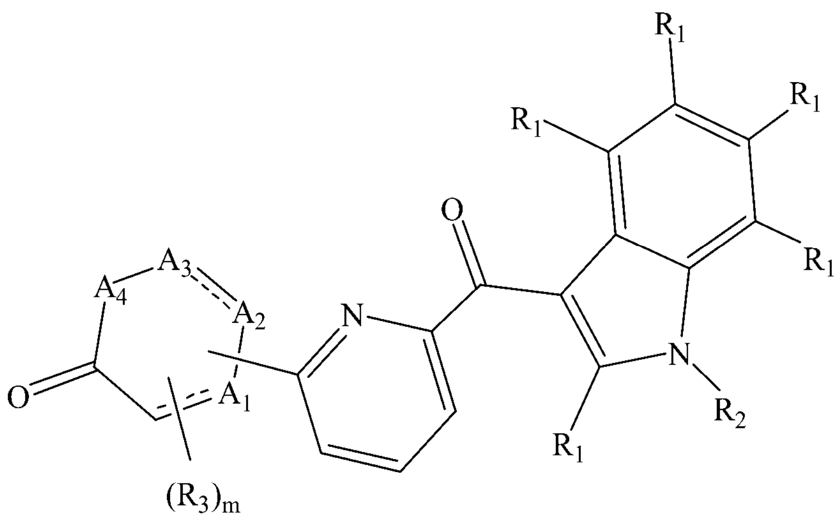

(Ib).

**5.** The aryl hydrocarbon receptor modulator according to claim 4, wherein

when $A_4$ is O, the general formula (Ib) is a general formula (Ib1):

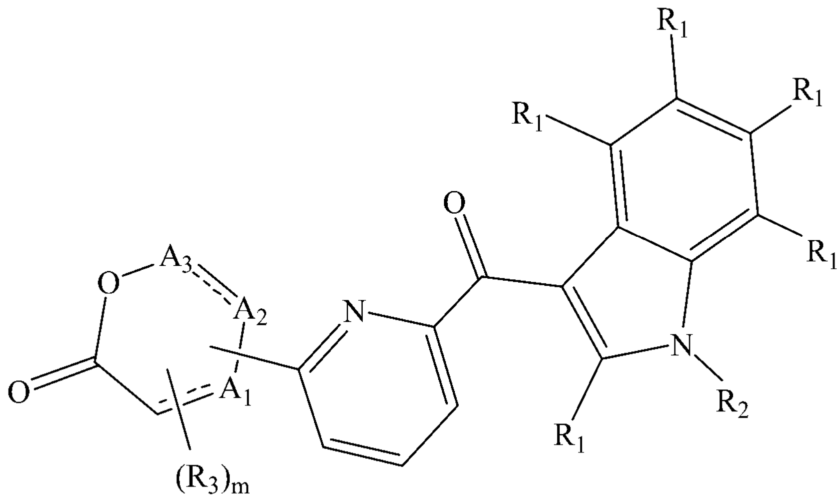

(Ib1);

or

when $A_4$ is $NR_4$, the general formula (Ib) is a general formula (Ib2):

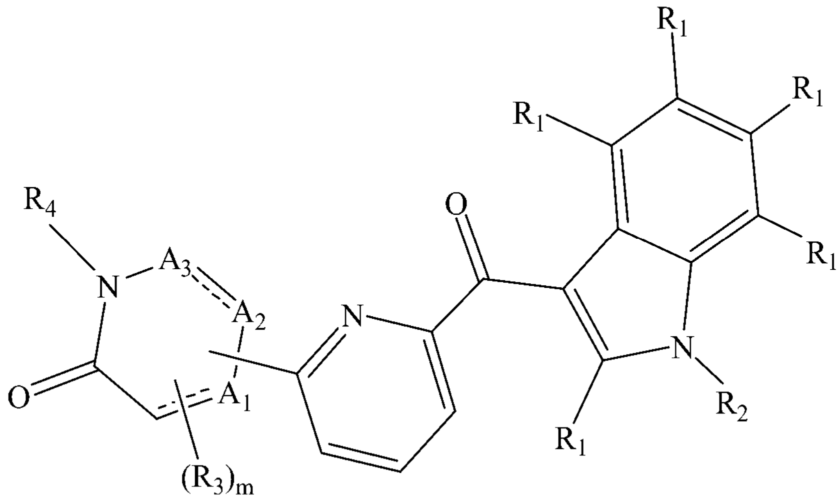

(Ib2).

**6.** The aryl hydrocarbon receptor modulator according to any one of claims 1 to 5, wherein

$A_1$, $A_2$, and $A_3$ are each independently a bond or C; m is an integer from 1 to 5; R3 is independently H, D, halogen, CN, OR, SR, N(R)R, -C(O)R, -C(O)OR, RC(O)O-, $S(O)_nR$, unsubstituted C1-C6 alkyl or substituted C1-C6 alkyl with 1-3 $R_a$, unsubstituted C2-C6 alkenyl OR substituted C2-C6 alkenyl with 1-3 $R_a$, unsubstituted C2-C6 alkynyl or substituted C2-C6 alkynyl with 1-3 $R_a$, or unsubstituted C3-C8 cycloalkyl or substituted C3-C8 cycloalkyl with 1-3 $R_a$; and $R_a$ is D, halogen, OR, SR, N(R)R, -C(O)R, -C(O)OR, RC(O)O-, or $S(O)_nR$;
n is 1 or 2; and
R is independently H, C1-C6 alkyl, C2-C6 alkenyl, C2-C6 alkynyl, or C3-C8 cycloalkyl;
preferably,
$A_1$, $A_2$, and $A_3$ are each independently a bond or C; m is an integer from 1 to 3; $R_3$ is independently H, D, halogen, CN, OR, SR, N(R)R, -C(O)R, -C(O)OR, RC(O)O-, $S(O)_nR$, unsubstituted C1-C4 alkyl or substituted C1-C4 alkyl with 1-2 $R_a$, unsubstituted C2-C4 alkenyl or substituted C2-C4 alkenyl with 1-2 $R_a$, unsubstituted C2-C4 alkynyl or substituted C2-C4 alkynyl with 1-2 $R_a$, or unsubstituted C3-C7 cycloalkyl orsubstituted C3-C7 cycloalkyl with 1-2 $R_a$; and $R_a$ is D, halogen, OR, SR, N(R)R, -C(O)R, -C(O)OR, RC(O)O-, or $S(O)_nR$;
n is 1 or 2; and
R is independently H, C1-C4 alkyl, C2-C4 alkenyl, C2-C4 alkynyl, or C3-C7 cycloalkyl;
more preferably,
$A_1$, $A_2$, and $A_3$ are each independently a bond or C; m is an integer from 1 to 2; $R_3$ is independently H, D, halogen, CN, OR, SR, N(R)R, -C(O)R, -C(O)OR, RC(O)O-, $S(O)_nR$, unsubstituted C1-C3 alkyl or substituted C1-C3 alkyl

with 1 $R_a$, unsubstituted C2-C3 alkenyl or substituted C2-C3 alkenyl with 1 $R_a$, unsubstituted C2-C3 alkynyl or substituted C2-C3 alkynyl with 1 $R_a$, or unsubstituted C5-C6 cycloalkyl or substituted C5-C6 cycloalkyl with 1 $R_a$; and $R_a$ is D, halogen, OR, SR, N(R)R, -C(O)R, -C(O)OR, RC(O)O-, or $S(O)_nR$;
n is 1 or 2; and
R is independently H, C1-C3 alkyl, C2-C3 alkenyl, C2-C3 alkynyl, or C5-C6 cycloalkyl.

**7.** The aryl hydrocarbon receptor modulator according to any one of claims 1 to 5, wherein

$R_1$ is independently H, D, halogen, CN, OR, SR, N(R)R, -C(O)R, -C(O)OR, RC(O)O-, $S(O)_nR$, unsubstituted C1-C6 alkyl or substituted C1-C6 alkyl with 1-3 $R_b$, unsubstituted C2-C6 alkenyl or substituted C2-C6 alkenyl with 1-3 $R_b$, unsubstituted C2-C6 alkynyl or substituted C2-C6 alkynyl with 1-3 $R_b$, or unsubstituted C3-C8 cycloalkyl or substituted C3-C8 cycloalkyl with 1-3 $R_b$; $R_b$ is D, halogen, OR, SR, N(R)R, -C(O)R, -C(O)OR, RC(O)O-, or $S(O)_nR$; or two adjacent or meta $R_1$ groups form a 3-8 membered carbocycle or a 3-8 membered heterocycle containing 1-3 heteroatoms selected from N, O, or S;
n is 1 or 2; and
R is independently H, C1-C6 alkyl, C2-C6 alkenyl, C2-C6 alkynyl, or C3-C8 cycloalkyl;
preferably,
$R_1$ is independently H, D, halogen, CN, OR, SR, N(R)R, -C(O)R, -C(O)OR, RC(O)O-, $S(O)_nR$, unsubstituted C1-C4 alkyl or substituted C1-C4 alkyl with 1-2 $R_b$, unsubstituted C2-C4 alkenyl or substituted C2-C4 alkenyl with 1-2 $R_b$, unsubstituted C2-C4 alkynyl or substituted C2-C4 alkynyl with 1-2 $R_b$, or unsubstituted C3-C7 cycloalkyl or substituted C3-C7 cycloalkyl with 1-2 $R_b$; $R_b$ is D, halogen, OR, SR, N(R)R, -C(O)R, -C(O)OR, RC(O)O-, or $S(O)_nR$; or two adjacent or meta $R_1$ groups form a 3-7 membered carbocycle or a 3-7 membered heterocycle containing 1-2 heteroatoms selected from N, O, or S;
n is 1 or 2; and
R is independently H, C1-C4 alkyl, C2-C4 alkenyl, C2-C4 alkynyl, or C3-C7 cycloalkyl;
more preferably,
$R_1$ is independently H, D, halogen, CN, OR, SR, N(R)R, -C(O)R, -C(O)OR, RC(O)O-, $S(O)_nR$, unsubstituted C1-C3 alkyl or substituted C1-C3 alkyl with 1 $R_b$, unsubstituted C2-C3 alkenyl or substituted C2-C3 alkenyl with 1 $R_b$, unsubstituted C2-C3 alkynyl or substituted C2-C3 alkynyl with 1 $R_b$, or unsubstituted C5-C6 cycloalkyl or substituted C5-C6 cycloalkyl with 1 $R_b$; $R_b$ is D, halogen, OR, SR, N(R)R, -C(O)R, -C(O)OR, RC(O)O-, or $S(O)_nR$; or two adjacent or meta $R_1$ groups form a 5-6 membered carbocycle or a 5-6 membered heterocycle containing 1-2 heteroatoms selected from N, O, or S;
n is 1 or 2; and
R is independently H, C1-C3 alkyl, C2-C3 alkenyl, C2-C3 alkynyl, or C5-C6 cycloalkyl.

**8.** The aryl hydrocarbon receptor modulator according to any one of claims 1 to 5, wherein

$R_2$ is H, $-(CH_2O)_aC(O)(CH_2)_b(O)_c(CH(R_c))_d(CH_2CH_2O)_eR_c$, $-(CH_2O)_aP(O)(R_p)_2$, or - $(CH_2O)_a(CH(R_c))_d(CH_2CH_2O)_eR_c$, $R_p$ is independently selected from $(CH_2)_b(O)_c(CH(R_c))_d(CH_2CH_2O)_eR_c$; $R_c$ is independently H, D, C1-C6 alkyl, $NH_2$-, (C1-C6 alkyl)NH-, $(C1\text{-}C6\ alkyl)_2N$-, $NH_2$(C1-C6 alkyl)-, OH, HO-(C1-C6 alkyl)-, or (C1-C6 alkyl)O-; a, b, c, and d are each independently 0 or 1; and e is an integer from 0 to 2;
preferably,
$R_2$ is H, $-(CH_2O)_aC(O)(CH_2)_b(O)_c(CH(R_c))_d(CH_2CH_2O)_eR_c$, $-(CH_2O)_aP(O)(R_p)_2$, or - $(CH_2O)_a(CH(R_c))_d(CH_2CH_2O)_eR_c$; $R_p$ is independently selected from $(CH_2)_b(O)_c(CH(R_c))_d(CH_2CH_2O)_eR_c$; $R_c$ is independently H, D, C1-C4 alkyl, $NH_2$-, (C1-C4 alkyl)NH-, $(C1\text{-}C4\ alkyl)_2N$-, $NH_2$(C1-C4)alkyl-, OH, HO-(C1-C4)alkyl-, or (C1-C4 alkyl)O-; a, b, c, and d are each independently 0 or 1; and e is an integer of 0 or 1;
more preferably,
$R_2$ is H, $-(CH_2O)_aC(O)(CH_2)_b(O)_c(CH(R_c))_d(CH_2CH_2O)_eR_c$, $-(CH_2O)_aP(O)(R_p)_2$, or - $(CH_2O)_a(CH(R_c))_d(CH_2CH_2O)_eR_c$; $R_p$ is independently selected from $(CH_2)_b(O)_c(CH(R_c))_d(CH_2CH_2O)_eR_c$; $R_c$ is independently H, D, C1-C3 alkyl, $NH_2$-, (C1-C3 alkyl)NH-, $(C1\text{-}C3\ alkyl)_2N$-, $NH_2$(C1-C3)alkyl-, OH, HO-(C1-C3)alkyl-, or (C1-C3 alkyl)O-; a, b, c, and d are each independently 0 or 1; and e is an integer of 0 or 1.

**9.** The aryl hydrocarbon receptor modulator according to any one of claims 1 to 5, wherein

$R_4$ is H, unsubstituted C1-C6 alkyl or substituted C1-C6 alkyl with 1-3 $R_b$, unsubstituted C2-C6 alkenyl or substituted C2-C6 alkenyl with 1-3 $R_b$, unsubstituted C2-C6 alkynyl or substituted C2-C6 alkynyl with 1-3 $R_b$, or unsubstituted C3-C8 cycloalkyl or substituted C3-C8 cycloalkyl with 1-3 $R_b$; and $R_b$ is D, halogen, OR, SR, N(R)R, -C(O)R, -C(O)OR, RC(O)O-, or S(O)nR; and

R is independently H, C1-C6 alkyl, C2-C6 alkenyl, C2-C6 alkynyl, or C3-C8 cycloalkyl;
preferably,
$R_4$ is H, unsubstituted C1-C4 alkyl or substituted C1-C4 alkyl with 1-2 $R_b$, unsubstituted C2-C4 alkenyl or substituted C2-C4 alkenyl with 1-2 $R_b$, unsubstituted C2-C4 alkynyl or substituted C2-C4 alkynyl with 1-2 $R_b$, or unsubstituted C3-C7 cycloalkyl or substituted C3-C7 cycloalkyl with 1-2 $R_b$; and $R_b$ is D, halogen, OR, SR, N(R)R, -C(O)R, -C(O)OR, RC(O)O-, or $S(O)_nR$; and
R is independently H, C1-C4 alkyl, C2-C4 alkenyl, C2-C4 alkynyl, or C3-C7 cycloalkyl;
more preferably,
$R_4$ is H, unsubstituted C1-C3 alkyl or substituted C1-C3 alkyl with 1 $R_b$, unsubstituted C2-C3 alkenyl or substituted C2-C3 alkenyl with 1 $R_b$, unsubstituted C2-C3 alkynyl or substituted C2-C3 alkynyl with 1 $R_b$, or unsubstituted C5-C6 cycloalkyl or substituted C5-C6 cycloalkyl with 1 $R_b$; and $R_b$ is D, halogen, OR, SR, N(R)R, -C(O)R, -C(O)OR, RC(O)O-, or $S(O)_nR$; and
R is independently H, C l-C3 alkyl, C2-C3 alkenyl, C2-C3 alkynyl, or C5-C6 cycloalkyl.

**10.** The aryl hydrocarbon receptor modulator according to any one of claims 1 to 5, wherein the aryl hydrocarbon receptor modulator is the following compounds:

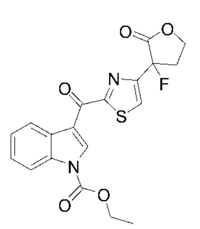 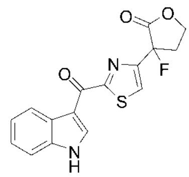 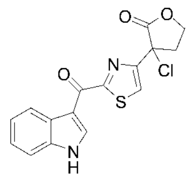 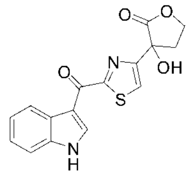 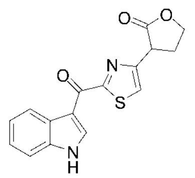

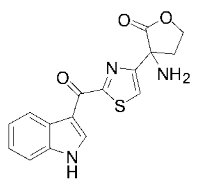 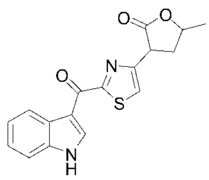 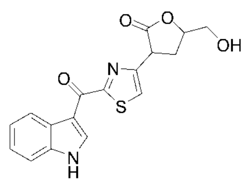 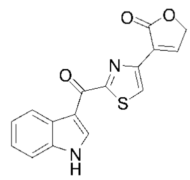 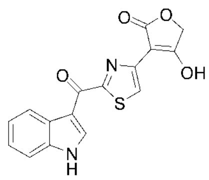

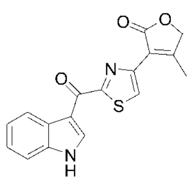 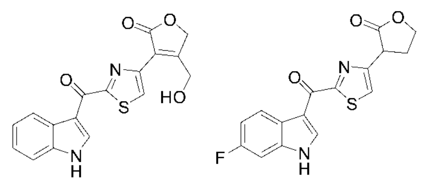 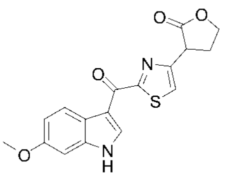 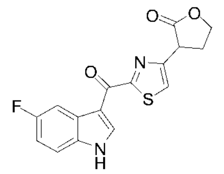

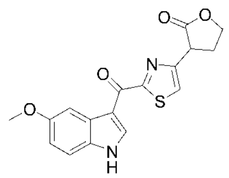 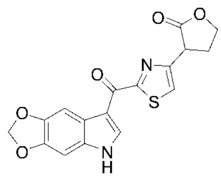 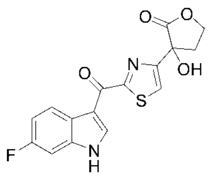 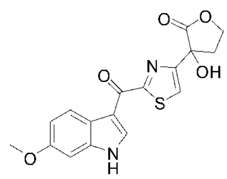 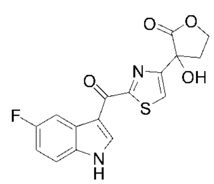

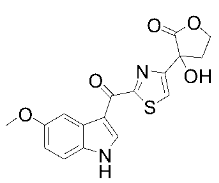 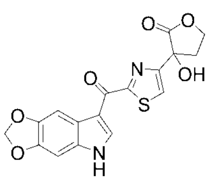 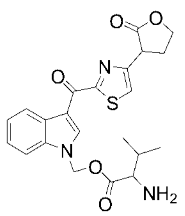 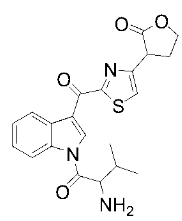 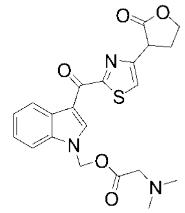

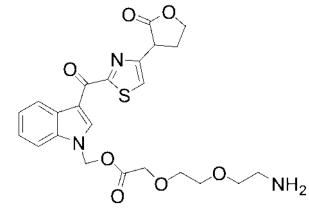
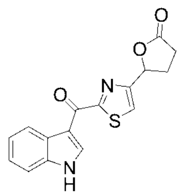
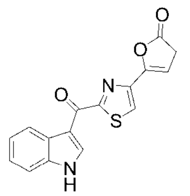
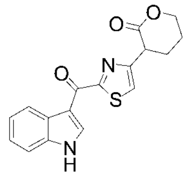
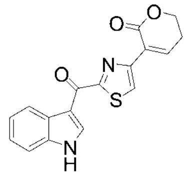

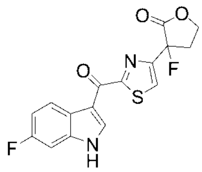
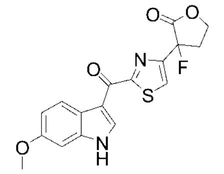
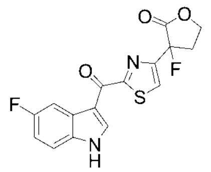
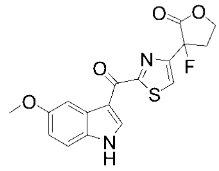
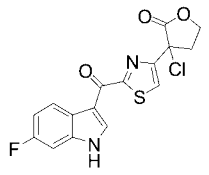

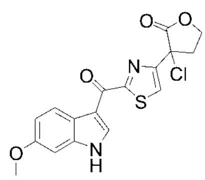
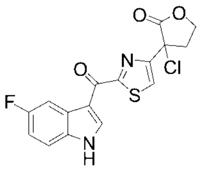
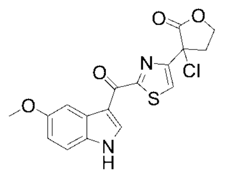
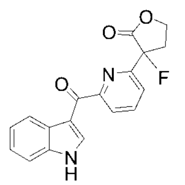
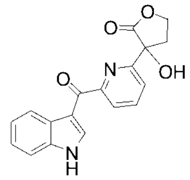

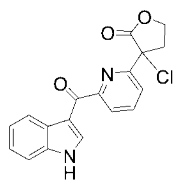
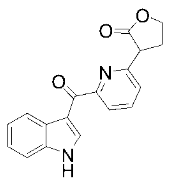
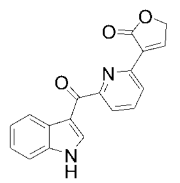
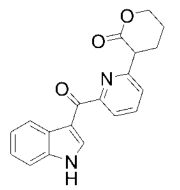
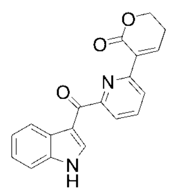

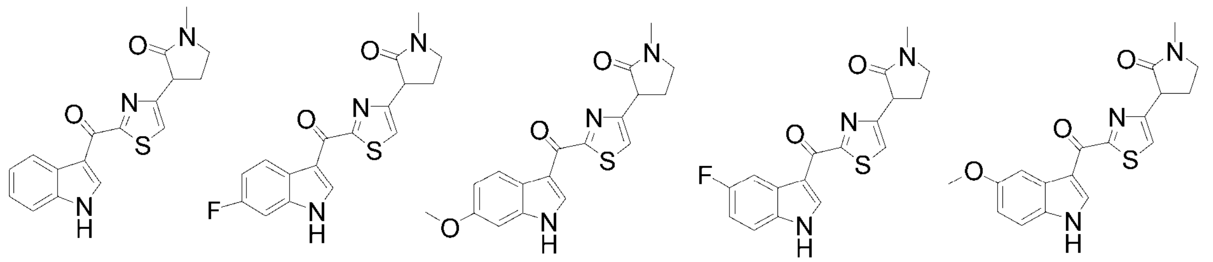

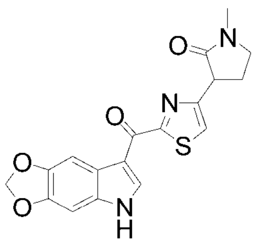
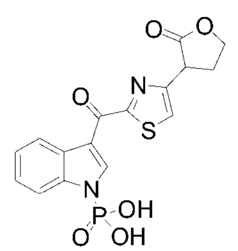
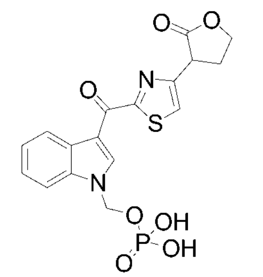
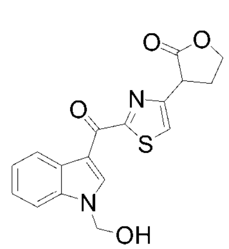

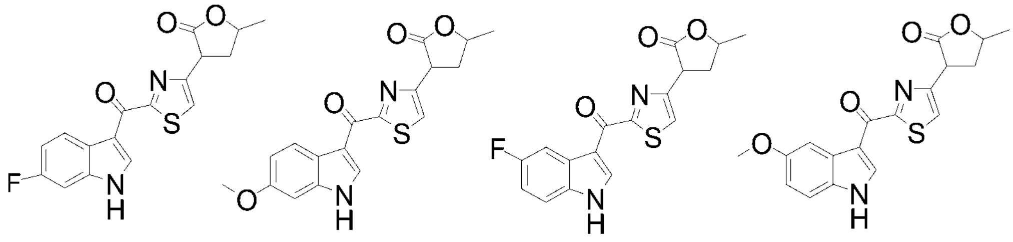

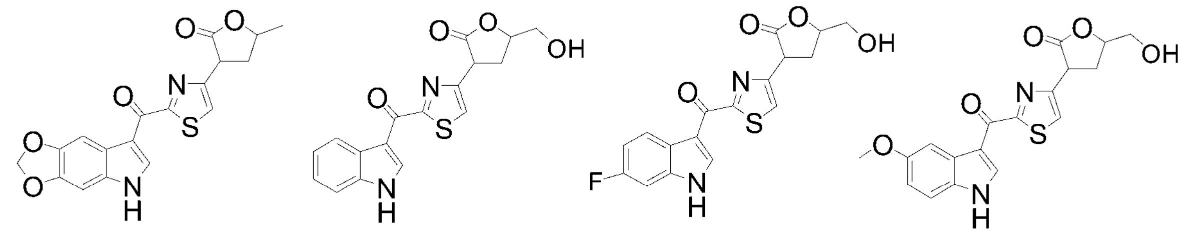

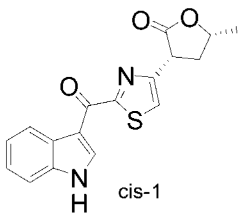
cis-1
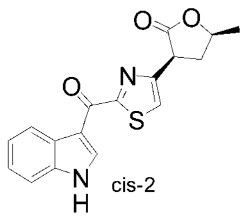
cis-2
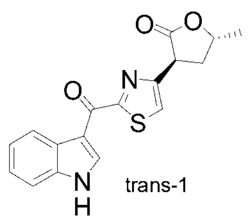
trans-1
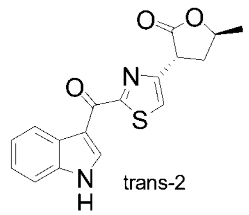
trans-2

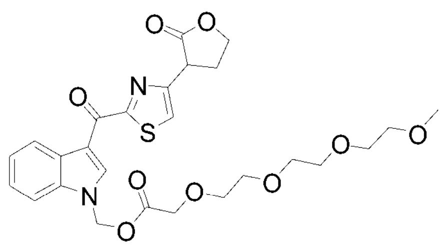

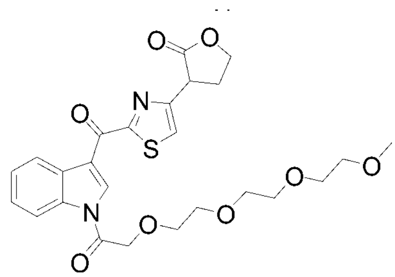
.

**11.** A salt, a prodrug, a hydrate, a solvate, or a deuterated derivative further substituted with deuterium of the aryl hydrocarbon receptor modulator according to any one of claims 1 to 5.

**12.** A use of the aryl hydrocarbon receptor modulator according to claim 1 in the preparation of a medicament for treating a central nervous system disease, cancer, or obesity, or in the preparation of a medicament for immunomodulation, hematopoiesis, cell cycle regulation, or intestinal barrier regulation; preferably, the central nervous system disease is selected from Alzheimer's disease, Parkinson's disease, or multiple sclerosis; the immunomodulation is selected from immunomodulation for psoriasis, atopic dermatitis, lupus erythematosus, or vitiligo; and the intestinal barrier regulation is for inflammatory bowel disease.

**13.** A use of the aryl hydrocarbon receptor modulator according to claim 1 in the treatment of a central nervous system disease, cancer, or obesity, or in immunomodulation, hematopoiesis, cell cycle regulation, or intestinal barrier regulation; preferably, the central nervous system disease is selected from Alzheimer's disease, Parkinson's disease, or multiple sclerosis; the immunomodulation is selected from immunomodulation for psoriasis, atopic dermatitis, lupus erythematosus, or vitiligo; and the intestinal barrier regulation is for inflammatory bowel disease.

**INTERNATIONAL SEARCH REPORT**

International application No.
**PCT/CN2024/118483**

**A. CLASSIFICATION OF SUBJECT MATTER**

C07D417/14(2006.01)i; C07D419/14(2006.01)i; C07D417/06(2006.01)i; C07D403/14(2006.01)i; A61K31/404(2006.01)i; A61K31/426(2006.01)i; A61K31/4427(2006.01)i; A61P35/00(2006.01)i

According to International Patent Classification (IPC) or to both national classification and IPC

**B. FIELDS SEARCHED**

Minimum documentation searched (classification system followed by classification symbols)

IPC:C07D A61K A61P

Documentation searched other than minimum documentation to the extent that such documents are included in the fields searched

Electronic data base consulted during the international search (name of data base and, where practicable, search terms used)

CNABS; WPABS; DWPI; VEN; CNKI; CJFD; CNTXT; USTXT; WOTXT; EPTXT; STN-REGISTRY; STN-CAPLUS; 万方, Wanfang; 超星读秀, CHAOXING DUXIU; ISI-Web of Science: 德明药泰, 张所明, 芳香烃受体, 吲哚, 噻唑, 内酰胺, 内酯, 癌症, 肿瘤, AHR, +indole+, +thiazole+, cancer, tumo?r, 结构式检索, structural formula search

**C. DOCUMENTS CONSIDERED TO BE RELEVANT**

| Category* | Citation of document, with indication, where appropriate, of the relevant passages | Relevant to claim No. |
|---|---|---|
| A | WO 2018121434 A1 (SHANGHAI ZHENGJI PHARMACEUTICAL TECHNOLOGY CO., LTD.) 05 July 2018 (2018-07-05)<br>claim 25 | 1-12 |
| A | US 2023065463 A1 (FOGHORN THERAPEUTICS INC.) 02 March 2023 (2023-03-02)<br>entire document | 1-12 |
| A | WO 2017123884 A1 (ENANTA PHARM INC.) 20 July 2017 (2017-07-20)<br>entire document | 1-12 |
| A | WO 2020106695 A1 (ARIAGEN, INC.) 28 May 2020 (2020-05-28)<br>entire document | 1-12 |
| A | WO 2020160193 A2 (FOGHORN THERAPEUTICS INC.) 06 August 2020 (2020-08-06)<br>entire document | 1-12 |
| A | WO 2020214740 A1 (ARIAGEN, INC.) 22 October 2020 (2020-10-22)<br>entire document | 1-12 |

☐ Further documents are listed in the continuation of Box C.  ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **26 November 2024** | **10 December 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2024/118483**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **13**
because they relate to subject matter not required to be searched by this Authority, namely:

Claim 13 relates to the use of the aromatic hydrocarbon receptor modulator of claim 1 in the treatment of central nervous system diseases, cancers and obesity or in the aspect of immunoregulation, hematopoiesis, cell cycles or intestinal barrier, which is a disease treatment method that is practiced on a living human or animal body for the direct purpose of disease treatment, falls within methods for treatment of the human or animal body by therapy, and falls within the cases set out in PCT Rule 39.1(iv) for which no international search is required.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2024/118483**

| Patent document cited in search report | Publication date (day/month/year) | Patent family member(s) | Publication date (day/month/year) |
|---|---|---|---|
| WO 2018121434 A1 | 05 July 2018 | WO 2019123007 A1 | 27 June 2019 |
| | | KR 20220017518 A | 11 February 2022 |
| | | KR 102536298 B1 | 30 May 2023 |
| | | AU 2021200452 A1 | 25 February 2021 |
| | | AU 2021200452 B2 | 08 September 2022 |
| | | JP 2022024049 A | 08 February 2022 |
| | | KR 20190093198 A | 08 August 2019 |
| | | EP 3564239 A1 | 06 November 2019 |
| | | EP 3564239 A4 | 09 September 2020 |
| | | EP 3564239 B1 | 03 August 2022 |
| | | EP 3564239 B9 | 19 April 2023 |
| | | US 2019307731 A1 | 10 October 2019 |
| | | AU 2017389794 A1 | 27 June 2019 |
| | | AU 2017389794 B2 | 18 February 2021 |
| | | US 2019330201 A1 | 31 October 2019 |
| | | US 11547698 B2 | 10 January 2023 |
| | | JP 2020503385 A | 30 January 2020 |
| | | CN 108239083 A | 03 July 2018 |
| | | CN 108239083 B | 17 August 2021 |
| | | CN 113480530 A | 08 October 2021 |
| | | HK 40029475 A0 | 19 February 2021 |
| US 2023065463 A1 | 02 March 2023 | EP 3917934 A2 | 08 December 2021 |
| | | EP 3917934 A4 | 15 February 2023 |
| | | WO 2020160193 A2 | 06 August 2020 |
| | | WO 2020160193 A3 | 10 September 2020 |
| WO 2017123884 A1 | 20 July 2017 | WO 2017123884 A8 | 22 February 2018 |
| | | US 2019040084 A1 | 07 February 2019 |
| | | US 10759816 B2 | 01 September 2020 |
| | | EP 3402799 A1 | 21 November 2018 |
| | | EP 3402799 A4 | 07 August 2019 |
| | | EP 3402799 B1 | 04 May 2022 |
| | | US 2017226129 A1 | 10 August 2017 |
| | | CN 108699077 A | 23 October 2018 |
| | | HK 1260101 A0 | 13 December 2019 |
| | | CN 108699077 B | 02 March 2021 |
| WO 2020106695 A1 | 28 May 2020 | EP 3883605 A1 | 29 September 2021 |
| | | TW 202038943 A | 01 November 2020 |
| | | US 2023054194 A1 | 23 February 2023 |
| WO 2020160193 A2 | 06 August 2020 | EP 3917934 A2 | 08 December 2021 |
| | | EP 3917934 A4 | 15 February 2023 |
| | | WO 2020160193 A3 | 10 September 2020 |
| | | US 2023065463 A1 | 02 March 2023 |
| WO 2020214740 A1 | 22 October 2020 | AU 2020258394 A1 | 28 October 2021 |
| | | US 2021188834 A1 | 24 June 2021 |
| | | US 11390621 B2 | 19 July 2022 |
| | | CA 3137027 A1 | 22 October 2020 |
| | | US 2022389002 A1 | 08 December 2022 |
| | | TW 202104218 A | 01 February 2021 |
| | | EP 3956327 A1 | 23 February 2022 |

Form PCT/ISA/210 (patent family annex) (July 2022)

**INTERNATIONAL SEARCH REPORT**
**Information on patent family members**

International application No.
**PCT/CN2024/118483**

| Patent document cited in search report | Publication date (day/month/year) | Patent family member(s) | | | Publication date (day/month/year) |
|---|---|---|---|---|---|
| | | JP | 2022528977 | A | 16 June 2022 |
| | | KR | 20210151951 | A | 14 December 2021 |
| | | IL | 287177 | A | 01 December 2021 |
| | | WO | 2020214740 | A9 | 26 November 2020 |
| | | MX | 2021012543 | A | 10 December 2021 |
| | | CN | 113906021 | A | 07 January 2022 |
| | | IN | 202117045315 | A | 21 January 2022 |
| | | HK | 40059599 | A0 | 06 May 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **MURRAY et al.** *Nat. Rev. Cancer*, 2014, vol. 14 (12), 801-814
- **BERSTEN et al.** *Nat. Rev. Cancer*, 2013, vol. 13 (12), 827-841
- **QUINTANA et al.** *Nature*, 2008, vol. 453 (7191), 65-71
- **MEZRICH et al.** *J. Immunol.*, 2010, vol. 185 (6), 3190-3198
- **KIMURA et al.** *J. Exp. Med.*, 2009, vol. 206 (9), 2027-2035
- **WANG et al.** *Clin. Exp. Immunol.*, 2014, vol. 177 (2), 521-530
- **WEI et al.** *Lab. Invest.*, 2014, vol. 94 (5), 528-535
- **NGUYEN et al.** *Proc. Natl. Acad. Sci. USA*, 2010, vol. 107 (46), 19961-19966